(19) Europäisches Patentamt European Patent Office Office européen des brevets

(11) **EP 3 500 550 B1**

(12) **EUROPÄISCHE PATENTSCHRIFT**

(45) Veröffentlichungstag und Bekanntmachung des Hinweises auf die Patenterteilung:
**17.03.2021 Patentblatt 2021/11**

(21) Anmeldenummer: **17727116.0**

(22) Anmeldetag: **15.05.2017**

(51) Int Cl.:
*C07C 69/675* (2006.01)    *A61K 8/37* (2006.01)
*A61Q 19/00* (2006.01)    *B01F 17/00* (2006.01)

(86) Internationale Anmeldenummer:
**PCT/EP2017/061563**

(87) Internationale Veröffentlichungsnummer:
**WO 2018/033259 (22.02.2018 Gazette 2018/08)**

(54) **VERNETZTE POLYGLYCERINESTER**

CROSSLINKED POLYGLYCEROL ESTERS

ESTERS DE POLYGLYCÉRINE RÉTICULÉS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **18.08.2016 EP 16184676**

(43) Veröffentlichungstag der Anmeldung:
**26.06.2019 Patentblatt 2019/26**

(73) Patentinhaber: **Evonik Operations GmbH**
**45128 Essen (DE)**

(72) Erfinder:
• **VON HOF, Jan Marian**
**44789 Bochum (DE)**
• **FRIEDRICH, Achim**
**45529 Hattingen (DE)**
• **BERKELS, Wolfgang**
**46238 Bottrop (DE)**
• **HOHENBERG, Barbara**
**45896 Gelsenkirchen (DE)**
• **SPRINGER, Oliver**
**46485 Wesel (DE)**
• **MEYER, Juergen**
**45259 Essen (DE)**

(74) Vertreter: **Evonik Patent Association**
**c/o Evonik Industries AG**
**IP Management**
**Bau 1042A/PB 15**
**Paul-Baumann-Straße 1**
**45772 Marl (DE)**

(56) Entgegenhaltungen:
**EP-A1- 0 835 862**    **EP-A2- 1 500 427**
**EP-A2- 1 683 781**

**Beschreibung**

Gebiet der Erfindung

Gegenstand der Erfindung ist ein Polyglycerinpartialester erhältlich durch Veresterung eines Polyglycerins mit einer Carbonsäuremischung umfassend mindestens eine Polyhydroxycarbonsäure einer Hydroxycarbonsäure aufweisend 8 bis 32 Kohlenstoffatome, mindestens eine kurzkettige Dicarbonsäure aufweisend 2 bis 16, bevorzugt 6 bis 14, besonders bevorzugt 8 bis 12, Kohlenstoffatome, mindestens eine langkettige Dicarbonsäure aufweisend 24 bis 44, bevorzugt 30 bis 40, besonders bevorzugt 34 bis 38, Kohlenstoffatome, und mindestens eine Fettsäure ausgewählt aus linearen, ungesättigten und verzweigten, gesättigten Fettsäuren aufweisend 14 bis 24 Kohlenstoffatome, Verfahren zur Herstellung von Polyglycerinpartialestern, sowie die Verwendung entsprechender Polyglycerinpartialester als W/O-Emulgator.

Stand der Technik

Die EP0835862 beschreibt Polyglycerinpartialester von gesättigten oder ungesättigten, linearen oder verzweigten Fettsäuren und mehrfunktionellen Carbonsäuren, erhältlich durch Veresterung eines Polyglyceringemisches mit gesättigten oder ungesättigten, linearen oder verzweigten Fettsäuren mit 12 bis 22 C-Atomen und mehrfunktionellen Carbonsäuren mit 4 bis 54 C-Atomen und einer mittleren Funktionalität von 2 bis 2,4, wobei der Veresterungsgrad der Polyglycerinmischung zwischen 30 und 75 % liegt. Das Polyglycerin weist bevorzugt einen mittleren Kondensationsgrad n von $\geq 2$, bevorzugt 3 bis 4, auf. Als mehrfunktionelle Carbonsäuren werden bevorzugt langkettige Dimersäuren, die durch katalysierte Dimerisierung ungesättigter Fettsäuren mit 12 bis 22 C-Atomen erhalten werden, eingesetzt. Polyhydroxyfettsäuren kommen nicht zum Einsatz.

Die EP1683781 beschreibt Polyglycerinpartialester von Polyricinolsäure und mehrfunktionellen Carbonsäuren, erhältlich durch Veresterung a) eines Polyglyceringemisches mit b) mindestens einer bestimmten Polyricinolsäure und gegebenenfalls b1) Polyhydroxystearinsäure und c) mindestens einer Di- und/oder Tricarbonsäure und d) mindestens einer Fettsäure nach an sich bekannten Verfahren. Das Polyglycerin weist bevorzugt einen mittleren Kondensationsgrad n von 1 bis 11, bevorzugt 2 bis 6, auf. Als Di- und/oder Tricarbonsäuren werden bevorzugt kurzkettige eingesetzt, als zusätzliche Fettsäurekomponente werden bevorzugt gesättigte Fettsäuren eingesetzt. In den Beispielen wird immer Sebacinsäure und Polyricinolsäure mit Polyglycerin umgesetzt.

Die EP1500427 beschreibt Polyglycerinpartialester von Polyhydroxystearinsäure und mehrfunktionellen Carbonsäuren, erhältlich durch Veresterung eines Polyglyceringemisches mit Polyhydroxystearinsäure und Di- und/oder Tricarbonsäuren und gegebenenfalls/oder mit Dimerfettsäuren und Fettsäuren mit 6 bis 22 C-Atomen. Das Polyglycerin weist bevorzugt einen mittleren Kondensationsgrad n von 1 bis 11, bevorzugt 2 bis 6, insbesondere bevorzugt 3 bis 6, auf. Als Di- und/oder Tricarbonsäuren werden bevorzugt Alkandicarbonsäuren mit 4 bis 14 C-Atomen eingesetzt. Die Schrift lehrt, dass zum Einsatzzweck als Emulgator der Einsatz von relativ kurzkettigen (C4 bis C14) Di- oder Tricarbonsäuren anstelle von langkettigen (>C24) Dimersäuren bevorzugt ist.

Ein Nachteil der im Stand der Technik beschriebenen Polyglycerinpartialester ist es, dass diese nicht in der Lage sind, besonders hohe Gehalte an natürlichen Ölen in W/O-Emulsionen zu stabilisieren. Außerdem ist es nicht möglich, einzig mit den im Stand der Technik beschriebenen PEG-freien Polyglycerinpartialestern, so beispielsweise mit denen der EP1683781, EP1500427 oder EP0835862, sogenannte *quick-breaking*-Emulsionen, wie sie beispielsweise in WO2014090615 oder EP2319484 beschrieben werden, zu stabilisieren.

Aufgabe der Erfindung war es, Polyglycerinpartialester mit einem im Vergleich zum Stand der Technik verbesserten Emulsionsstabilisierungsvermögen, insbesondere bei einem hohen Gehalt an Öl, zu entwickeln.

Beschreibung der Erfindung

Überraschenderweise wurde gefunden, dass die im Folgenden beschriebenen Polyglycerinpartialester die der Erfindung gestellte Aufgabe zu lösen vermögen.

Gegenstand der vorliegenden Erfindung ist daher ein Polyglycerinpartialester erhältlich durch Veresterung eines Polyglycerins mit einer Carbonsäuremischung umfassend:

    a) mindestens eine Polyhydroxycarbonsäure einer Hydroxycarbonsäure aufweisend 8 bis 32, bevorzugt 12 bis 22, besonders bevorzugt 14 bis 18, Kohlenstoffatome,
    b) mindestens eine kurzkettige Dicarbonsäure aufweisend 2 bis 16, bevorzugt 6 bis 14, besonders bevorzugt 8 bis 12, Kohlenstoffatome,
    c) mindestens eine langkettige Dicarbonsäure aufweisend 24 bis 44, bevorzugt 30 bis 40, besonders bevorzugt 34 bis 38, Kohlenstoffatome, und

d) mindestens eine Fettsäure ausgewählt aus linearen, ungesättigten und verzweigten, gesättigten Fettsäuren aufweisend 14 bis 24, bevorzugt 16 bis 20, Kohlenstoffatome.

Ein weiterer Gegenstand der Erfindung ist ein Verfahren zur Herstellung solcher Polyglycerinpartialester, sowie die Verwendung entsprechender Polyglycerinpartialester als W/O-Emulgator.

Vorteile sind:

Ein Vorteil der vorliegenden Erfindung ist es, dass sich mit den Polyglycerinpartialestern hervorragende *quick-breaking*-Systeme formulieren lassen, die als W/O-Emulsionen extrem hohe Mengen an interner Wasserphase, insbesondere bei extrem niedriger Emulgatorkonzentration aufweisen und beim Verteilen auf der Haut die interne Phase freigeben. Derartige *quick-breaking* Systeme können mit den erfindungsgemäßen Emulgatoren erstmals auch komplett basierend auf natürlichen Inhaltsstoffen hergestellt werden.

Ein weiterer Vorteil der vorliegenden Erfindung ist es, dass auch hohe Anteile an natürlichen Ölen wie beispielsweise Mandelöl in Emulsionen gut stabilisiert werden.

Ein weiterer Vorteil der vorliegenden Erfindung ist es, dass Emulsionen über einen breiten Viskositätsbereich von Sprays über Lotionen bis hin zu Cremes stabilisiert werden können.

Ein weiterer Vorteil vorliegenden Erfindung ist es, dass Emulsionen basierend auf erfindungsgemäßen Emulgatoren eine sehr gute Kompatibilität mit Treibgasen, wie beispielsweise Mischungen aus Propan, *n*-Butan und *iso*-Butan, aufweisen und somit die Herstellung von Aerosolsystemen vereinfachen.

Noch ein Vorteil der vorliegenden Erfindung ist es, dass die mit den beschriebenen Polyglycerinpartialestern stabilisierte Emulsionen PEG-frei formuliert werden können.

Ein weiterer Vorteil der vorliegenden Erfindung ist es, dass das Hautgefühl der mit den beschriebenen Polyglycerinpartialestern stabilisierten Emulsionen mindestens ebenso gut ist wie mit den Emulgatoren des Stands der Technik.

Noch ein Vorteil der vorliegenden Erfindung ist es, dass der Ölanteil der mit den beschriebenen Polyglycerinpartialestern stabilisierte Emulsionen schnell durch die Haut absorbiert wird.

Ein weiterer Vorteil der vorliegenden Erfindung ist es, dass Emulsionen basierend auf den erfindungsgemäßen Emulgatoren eine sehr hohe Elektrolyttoleranz, beispielsweise emulsionsstabil gegenüber 7 Gew.-% NaCl, aufweisen.

Die erfindungsgemäßen Polyglycerinpartialester stellen Mischungen von unterschiedlichen Substanzen dar; daher ist dem Fachmann klar, dass angegebene Zahlenwerte Mittelwerte über die Mischung darstellen können.

Unter dem Begriff "Polyglycerin" im Sinne der vorliegenden Erfindung ist ein Polyglycerin zu verstehen, welches auch Glycerin enthalten kann. Somit ist zur Berechnung von Mengen, Massen und dergleichen gegebenenfalls ein Glycerinanteil mit zu berücksichtigen. Polyglycerine im Sinne der vorliegenden Erfindung sind somit auch Mischungen enthaltend mindestens ein Glycerin-Oligomer und Glycerin. Unter Glycerin-Oligomeren sind jeweils alle entsprechenden Strukturen, z.B. also lineare, verzweigte und zyklische Verbindungen zu verstehen.

Analoges gilt für den Begriff "Polyglycerinpartialester" im Zusammenhang mit der vorliegenden Erfindung. Alle angegebenen Prozent (%) sind, wenn nicht anders angegeben, Massenprozent.

Erfindungsgemäß bevorzugter Polyglycerinpartialester ist dadurch gekennzeichnet, dass das eingesetzte Polyglycerin einen mittleren Kondensationsgrad von 1,5 bis 15, bevorzugt von 2 bis 6, besonders bevorzugt von 3 bis 5, aufweist.

Der mittlere Polymerisationsgrad $N$ des Polyglycerins wird über dessen Hydroxylzahl (OHZ, in mg KOH/g) gemäß folgender Formel berechnet:

$$N = \frac{(112200 - 18 \cdot OHZ)}{(74 \cdot OHZ - 56100)}$$

Geeignete Bestimmungsmethoden zur Ermittlung der Hydroxylzahl sind insbesondere solche gemäß DGF C-V 17 a (53), Ph. Eur. 2.5.3 Method A und DIN 53240.

Somit ist erfindungsgemäß bevorzugter Polyglycerinpartialester dadurch gekennzeichnet, dass das eingesetzte Polyglycerin eine Hydroxylzahl von 1520 bis 845, bevorzugt von 1350 bis 970, besonders bevorzugt von 1170 bis 1010 mg KOH/g, aufweist.

Das eingesetzte Polyglycerin kann durch verschiedene konventionelle Methoden wie beispielsweise Polymerisation von Glycidol (z.B. basenkatalysiert), Polymerisation von Epichlorhydrin (beispielsweise in Gegenwart äquimolarer Mengen einer Base wie NaOH) oder Polykondensation von Glycerin bereitgestellt werden.

Erfindungsgemäß bevorzugt ist die Bereitstellung des Polyglycerins durch die Kondensation von Glycerin, insbesondere in Gegenwart katalytischer Mengen einer Base, insbesondere NaOH oder KOH. Geeignete Reaktionsbedingungen sind Temperaturen zwischen 200-260 °C und reduzierter Druck in einem Bereich zwischen 20 bis 800 mbar, insbesondere zwischen 50 und 500 mbar, wodurch eine erleichterte Wasserentfernung möglich ist. Entsprechende Verfahren lassen sich Standardlehrwerken der Chemie wie beispielsweise dem Römpp entnehmen.

Es ist erfindungsgemäß bevorzugt, dass das eingesetzte Polyglycerin

0 bis 15% Glycerin,

10 bis 40% Diglycerin,

10 bis 50% Triglycerin,

5 bis 25% Tetraglycerin und

0 bis 55% Pentaglycerin und höhere Anteile

enthält.

Zur Bestimmung der Oligomerenverteilung mittels GC wird ein Aliquot des Polyglycerins in 2 ml Pyridin:Chloroform (4:1) gelöst. 0,5 ml dieser Lösung werden mit 1 ml MSTFA [N-Methyl-N-(trimethylsilyl) trifluoroacetamide] versetzt. Die Alkohole werden durch Reaktion bei 80°C (30 Minuten) quantitativ in ihre Trimethylsilyether überführt und anschließend mittels GC/FID untersucht.

Dieses wird in einem Gaschromatograph, welcher mit einem split/splitless Injektor, einer Kapillarsäule und einem Flammenionisationsdetektor ausgestattet ist, unter folgenden Bedingungen durchgeführt:

| Injektor: | 290 °C, Split 30 ml |
|---|---|
| Injektionsvolumen: | 1 $\mu$l |
| Säule: | 30 m *0,32 mm HP1 0,25 $\mu$m |
| Trägergas: | Helium, constant flow, 2 mL/min |
| Temperaturprogramm: | 80 °C - 300 °C mit 4 °C/min, dann 10 |
| | Minuten bei 300 °C konditionieren. |
| Detektor: | FID bei 310 °C |
| | Wasserstoff 35 ml/min |
| | Luft 240 ml/min |
| | Make Up Gas 12 ml/min |

Glycerin, Diglycerin, Triglycerin und Tetraglycerin werden aufgetrennt und ihr Massenanteil nach einer Methode des internen Standards bestimmt. Hierzu wird das GC System durch Vermessen von Mischungen der zu untersuchenden Glycerine und des internen Standards mit bekannter Zusammensetzung kalibriert, wobei Triglycerin und Tetraglycerin als Diglycerin ausgewertet werden. Pentaglycerin und höhere Anteile können durch Subtraktion der Glycerin-, Diglycerin-, Triglycerin- und Tetraglycerin-Anteile von 100% bestimmt werden.

Der erfindungsgemäße Polyglycerinpartialester ist erhältlich durch Veresterung eines Polyglycerins mit einer Carbonsäuremischung umfassend die Komponenten a), b), c) und d). Es ist erfindungsgemäß bevorzugt wenn die Komponenten a), b), c) und d) in Summe mindestens 80 Gew.-%, bevorzugt mindestens 90 Gew.-%, besonders bevorzugt mindestens 95 Gew.-%, bezogen auf die gesamte, eingesetzte Carbonsäuremischung ausmachen.

Erfindungsgemäß bevorzugter Polyglycerinpartialester ist dadurch gekennzeichnet, dass die Polyhydroxycarbonsäure ausgewählt ist aus Polyhydroxystearinsäure und Polyricinolsäure, wobei Polyhydroxystearinsäure besonders bevorzugt ist.

Erfindungsgemäß bevorzugter Polyglycerinpartialester ist dadurch gekennzeichnet, dass die Polyhydroxycarbonsäure einen mittleren Kondensationsgrad von 1,5 bis 9, bevorzugt von 2 bis 7, besonders bevorzugt von 3 bis 5, aufweist.

Der mittlere Kondensationsgrad der Polyhydroxycarbonsäure lässt sich folgendermaßen bestimmen:

| $$N = \dfrac{\dfrac{56106}{SZ} - 18.02}{M_{Monomer} - 18.02}$$ | mit | N = mittlerer Kondensationsgrad der Polyhydroxycarbonsäure |
|---|---|---|
| | | SZ = Säurezahl [mg KOH/g] |
| | | $M_{Monomer}$ = Molekulargewicht des Monomers [g/mol], z.B. 300,48 g/mol für Hydroxystearinsäure |

Geeignete Methoden zur Bestimmung der Säurezahl sind insbesondere solche gemäß DGF C-V 2, DIN EN ISO 2114, Ph.Eur. 2.5.1, ISO 3682 und ASTM D 974.

Geeignete Methoden zur Bestimmung der Iodzahl sind insbesondere solche gemäß DGF C-V 11 a (53) und Ph. Eur. 2.5.4 (Methode A).

Die Herstellung der erfindungsgemäß eingesetzten Polyhydroxycarbonsäuren erfolgt beispielsweise durch Polykondensation mindestens einer entsprechenden Hydroxycarbonsäure. Geeignete Reaktionsbedingungen sind Temperaturen zwischen 180-260 °C und Atmosphärendruck oder auch reduzierter Druck in einem Bereich zwischen 20 bis 800 mbar, insbesondere zwischen 50 und 500 mbar. Vorzugsweise wird hier Hydroxystearinsäure, insbesondere 12-Hydroxystearinsäure, die durch Härtung von Ricinolsäure bzw. technischer Ricinusölfettsäure gewonnen wird, eingesetzt. Es werden hier Polyhydroxystearinsäuren erhalten und bevorzugt eingesetzt, die vorzugsweise Säurezahlen zwischen 188 und 20, bevorzugt zwischen 97 und 33, besonders bevorzugt zwischen 65 und 40, aufweisen.

Es ist erfindungsgemäß bevorzugt, wenn die kurzkettige Dicarbonsäure im erfindungsgemäßen Polyglycerinpartialester ausgewählt ist aus aliphatischen, linearen Dicarbonsäuren, insbesondere Oxalsäure, Malonsäure, Bernsteinsäure, Glutarsäure, Adipinsäure, Pimelinsäure, Korksäure, Azelainsäure, Sebacinsäure, Dodecandisäure, wobei Sebacinsäure besonders bevorzugt ist.

Erfindungsgemäß bevorzugter Polyglycerinpartialester ist dadurch gekennzeichnet, dass die langkettige Dicarbonsäure ausgewählt ist aus solchen, die aus der Dimerisierung von Ölsäure und/oder Linolsäure erhältlich sind. Die aus solch einem Prozess als Dimerfettsäuren bekannten erhältlichen Mischungen können neben den langkettige acyclischen und cyclischen Dicarbonsäuren in untergeordnetem Ausmaß polymere Fettsäuren (trimer- und höher funktionelle) enthalten, welche wiederum zu dem Anteil der eingesetzten Carbonsäuremischung beitragen, die nicht durch die Komponenten a) bis d) umfasst wird. Die Funktionalität des Gemisches erhältlich aus der Dimerisierung von Ölsäure und/oder Linolsäure sollte bevorzugt im molaren Mittel einen Wert von 2,4 nicht überschreiten. Zur Herstellung und Verwendung von Dimerfettsäuren und deren physikalischen und chemischen Eigenschaften wird auch auf die Veröffentlichung "The Dimer Acids: The chemical and physicalproperties, reactions and applications", Ed. E.C. Leonard; Humko Sheffield Chemical, 1975, Memphis, Tenn., verwiesen.

Es ist erfindungsgemäß bevorzugt, wenn die mindestens eine Fettsäure ausgewählt aus linearen, ungesättigten und verzweigten, gesättigten Fettsäure im erfindungsgemäßen Polyglycerinpartialester ausgewählt ist aus Isostearinsäure, Undecylensäure, Myristoleinsäure, Palmitoleinsäure, Petroselinsäure, Ölsäure, Elaidinsäure, Vaccensäure, Gadoleinsäure, Icosensäure, Cetoleinsäure, Erucasäure, Nervonsäure, Linolsäure, Alpha-Linolensäure, Gamma-Linolensäure, Calendulasäure, Punicinsäure, Alpha-Elaeostearinsäure, Beta-Elaeostearinsäure, Arachidonsäure, Timnodonsäure, Clupanodonsäure, Cervonsäure, wobei Ölsäure und Isostearinsäure besonders bevorzugt sind.

Erfindungsgemäß bevorzugter Polyglycerinpartialester ist dadurch gekennzeichnet, dass der Veresterungsgrad von 35 bis 95 %, bevorzugt von 40 bis 90 %, besonders bevorzugt von 45 bis 85 % aller im Polyglycerinpartialester enthaltenen OH-Gruppen, einschließlich der in der Polyhydroxycarbonsäure enthaltenen, beträgt.

Der Veresterungsgrad aller OH-Gruppen lässt sich über die Hydroxylzahl, die Säurezahl und die Verseifungszahl nach folgender Formel bestimmen:

$$Veresterungsgrad = \frac{VZ - SZ}{VZ - SZ + OHZ} \cdot 100 \quad \text{mit} \quad \begin{array}{l} VZ = \text{Verseifungszahl} \\ SZ = \text{Säurezahl} \\ OHZ = \text{Hydroxylzahl} \end{array}$$

Geeignete Bestimmungsmethoden zur Ermittlung der Verseifungszahl sind insbesondere solche gemäß DGF C-V 3, DIN EN ISO 3681 und Ph.Eur. 2.5.6.

Erfindungsgemäß bevorzugter Polyglycerinpartialester ist dadurch gekennzeichnet, dass in der Veresterung pro Mol Polyglycerin

0,1 bis 2, bevorzugt 0,3 bis 1, besonders bevorzugt 0,4 bis 0,8, mol der Komponente a),
0,1 bis 2, bevorzugt 0,2 bis 0,9, besonders bevorzugt 0,3 bis 0,6, mol der Komponente b),
0,1 bis 2, bevorzugt 0,2 bis 0,9, besonders bevorzugt 0,2 bis 0,5, mol der Komponente c), und
0,5 bis 4, bevorzugt 0,8 bis 3, besonders bevorzugt 1,2 bis 2,2, mol der Komponente d) eingesetzt werden.

Erfindungsgemäß bevorzugter Polyglycerinpartialester ist dadurch gekennzeichnet, dass in der Veresterung das molare Verhältnis von b) zu c)

von 0,2 : 1,0 bis 1,0 : 0,2, bevorzugt
von 0,5 : 1,0 bis 1,0 : 0,5, besonders bevorzugt
von 0,7 : 1,0 bis 1,0 : 0,7,
beträgt.

Die Polyglycerinpartialester der vorliegenden Erfindung lassen sich durch klassische Veresterungsverfahren herstellen, wie sie beispielsweise in der EP0835862, EP1683781 und EP1500427 beschrieben werden. Bevorzugt werden die Polyglycerinpartialester der vorliegenden Erfindung mit dem im Folgenden beschriebenen erfindungsgemäßen Verfahren hergestellt.

Ein weiterer Gegenstand der vorliegenden Erfindung ist ein Verfahren zur Herstellung eines Polyglycerinpartialesters, umfassend

die Veresterung eines Polyglycerins mit

a) mindestens einer Polyhydroxycarbonsäure einer Hydroxycarbonsäure aufweisend 8 bis 32, bevorzugt 12 bis 22, besonders bevorzugt 14 bis 18, Kohlenstoffatome,

b) mindestens einer kurzkettige Dicarbonsäure aufweisend 2 bis 16, bevorzugt 6 bis 14, besonders bevorzugt 8 bis 12, Kohlenstoffatome,

c) mindestens einer langkettige Dicarbonsäure aufweisend 24 bis 44, bevorzugt 30 bis 40, besonders bevorzugt 34 bis 38, Kohlenstoffatome, und

d) mindestens einer Fettsäure ausgewählt aus linearen, ungesättigten und verzweigten, gesättigten Fettsäuren aufweisend 14 bis 24, bevorzugt 16 bis 20, Kohlenstoffatome,

bevorzugt bis zu einem Veresterungsgrad von 35 bis 95 %, bevorzugt von 40 bis 90 %, besonders bevorzugt von 45 bis 85 % aller im Polyglycerinpartialester enthaltenen OH-Gruppen, einschließlich der in der Polyhydroxycarbonsäure enthaltenen.

Die Veresterung der verschiedenen Komponenten a) bis d) mit dem Polyglycerin kann erfindungsgemäß gleichzeitig oder sequentiell in beliebiger Reihenfolge erfolgen, wobei gleichzeitig erfindungsgemäß bevorzugt ist.

In Verfahrensschritt d) wird erfindungsgemäß bevorzugt bis zu einer Säurezahl des erhaltenen Polyglycerinpartialesters im Bereich von 30 bis 0, bevorzugt von 15 bis 0, besonders bevorzugt von 5 bis 0, mg KOH/g verestert.

In bevorzugten Alternativen des erfindungsgemäßen Verfahrens umfasst das erfindungsgemäße Verfahren die Verfahrensschritte

A1) Veresterung eines Polyglycerins mit

a) mindestens einer Polyhydroxycarbonsäure einer Hydroxycarbonsäure aufweisend 8 bis 32, bevorzugt 12 bis 22, besonders bevorzugt 14 bis 18, Kohlenstoffatome und

d) mindestens einer Fettsäure ausgewählt aus linearen, ungesättigten und verzweigten, gesättigten Fettsäuren aufweisend 14 bis 24, bevorzugt 16 bis 20, Kohlenstoffatome,

bevorzugt bis zu einer Säurezahl des in diesem Schritt erhaltenen Polyglycerinpartialesters in einem Bereich von 30 bis 0, bevorzugt von 15 bis 0, besonders bevorzugt von 5 bis 0, mg KOH/g, und

B1) Veresterung des Verfahrensschrittproduktes aus A1) mit

b) mindestens einer kurzkettige Dicarbonsäure aufweisend 2 bis 16, bevorzugt 6 bis 14, besonders bevorzugt 8 bis 12, Kohlenstoffatome, und

c) mindestens einer langkettige Dicarbonsäure aufweisend 24 bis 44, bevorzugt 30 bis 40, besonders bevorzugt 34 bis 38, Kohlenstoffatome,

bevorzugt bis zu einer Säurezahl des in diesem Schritt erhaltenen Polyglycerinpartialesters in einem Bereich von 30 bis 0, bevorzugt von 15 bis 0, besonders bevorzugt von 5 bis 0, mg KOH/g, und

bevorzugt bis zu einem Veresterungsgrad von 35 bis 95 %, bevorzugt von 40 bis 90 %, besonders bevorzugt von 45 bis 85 % aller im Polyglycerinpartialester enthaltenen OH-Gruppen, einschließlich der in der Polyhydroxycarbonsäure enthaltenen

oder

A2) Veresterung eines Polyglycerins mit

b) mindestens einer kurzkettige Dicarbonsäure aufweisend 2 bis 16, bevorzugt 6 bis 14, besonders bevorzugt 8 bis 12, Kohlenstoffatome,

c) mindestens einer langkettige Dicarbonsäure aufweisend 24 bis 44, bevorzugt 30 bis 40, besonders bevorzugt 34 bis 38, Kohlenstoffatome, und

d) mindestens einer Fettsäure ausgewählt aus linearen, ungesättigten und verzweigten, gesättigten Fettsäuren aufweisend 14 bis 24, bevorzugt 16 bis 20, Kohlenstoffatome,

bevorzugt bis zu einer Säurezahl des in diesem Schritt erhaltenen Polyglycerinpartialesters in einem Bereich von 30 bis 0, bevorzugt von 15 bis 0, besonders bevorzugt von 5 bis 0, mg KOH/g, und

B2) Veresterung des Verfahrensschrittproduktes aus A2) mit

a) mindestens einer Polyhydroxycarbonsäure einer Hydroxycarbonsäure aufweisend 8 bis 32, bevorzugt 12 bis 22, besonders bevorzugt 14 bis 18, Kohlenstoffatome,

bevorzugt bis zu einer Säurezahl des in diesem Schritt erhaltenen Polyglycerinpartialesters in einem Bereich von 30 bis 0, bevorzugt von 15 bis 0, besonders bevorzugt von 5 bis 0, mg KOH/g, und

bevorzugt bis zu einem Veresterungsgrad von 35 bis 95 %, bevorzugt von 40 bis 90 %, besonders bevorzugt von 45 bis 85 % aller im Polyglycerinpartialester enthaltenen OH-Gruppen, einschließlich der in der Polyhydroxycarbonsäure enthaltenen.

Ein weiterer Gegenstand der vorliegenden Erfindung sind kosmetische oder pharmazeutische Zubereitungen enthaltend mindestens einen erfindungsgemäßen Polyglycerinpartialester und/oder einen Polyglycerinpartialester erhältlich nach dem erfindungsgemäßen Verfahren, insbesondere in einer Menge von 0,5 Gew.-% bis 20 Gew.-%, bevorzugt von 1 Gew.-% bis 10 Gew.-%, wobei sich die Gewichtsprozente auf die Gesamtzubereitung beziehen.

Bevorzugte erfindungsgenmäße Zubereitungen sind quick-break-Emulsionen. Schnellbrechende Wasser-in-Öl Emulsionen (sogenannte quick-break-Emulsionen) sind bekannt als Emulsionen mit einem hohen Wassergehalt, welche durch die bei der Anwendung auftretenden Scherkräfte sehr schnell brechen und dabei Wassertröpfchen freisetzen, die mitunter sichtbar sind.

Von daher enthalten bevorzugte erfindungsgemäße quick-break-Emulsionen eine interne wässrige Phase in einer Menge von 80 Gew.-% bis 94,5 Gew.-%, bevorzugt 83 Gew.-% bis 92 Gew.-%, eine externe Ölphase in einer Menge von 5 Gew.-% bis 20 Gew.-%, bevorzugt 8 Gew.-% bis 17 Gew.-%, und den mindestens einen erfindungsgemäßen Polyglycerinpartialester und/oder den einen Polyglycerinpartialester erhältlich nach dem erfindungsgemäßen Verfahren in einer Menge von 0,5 Gew.-% bis 2,0 Gew.-%, bevorzugt 0,5 Gew.-% bis 1,5 Gew.-%, wobei sich die Gewichtsprozente auf die Gesamtzubereitung beziehen.

Bevorzugt sind die erfindungsgemäßen Zubereitungen PEG-frei. Hierunter ist zu verstehen, dass erfindungsgemäß auf den Zusatz an Polyethylenglykolen und/oder Polyethylenglykolderivaten, wie beispielsweise ethoxylierten Ester, Fettsäure oder Fettalkohole, verzichtet werden kann. Der Anteil an PEG-haltigen Substanzen liegt daher unter 1 Gew.-% bezogen auf die Gesamtmasse der Zubereitung, um als erfindungsgemäß PEG-frei zu gelten. Bevorzugt liegt der Anteil an PEG-haltigen Substanzen bei 0 Gew.-%.

Ein weiterer Gegenstand der vorliegenden Erfindung ist die Verwendung mindestens eines erfindungsgemäßen Polyglycerinpartialesters und/oder eines Polyglycerinpartialesters erhältlich nach dem erfindungsgemäßen Verfahren als W/O-Emulgator, insbesondere in kosmetischen oder pharmazeutischen Zubereitungen sowie die Verwendung mindestens eines erfindungsgemäßen Polyglycerinpartialesters und/oder eines Polyglycerinpartialesters erhältlich nach dem erfindungsgemäßen Verfahren zur Herstellung von quick-break-Emulsionen.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.

Beispiele:

*Beispiel 1: PGE erfindungsgemäß*

Ein Gemisch aus Polyglycerin (OHZ = 1113 mg KOH/g, 52.3 g, 0.188 mol), Ölsäure (Palmera A1818 der Firma KLK Emmerich GmbH, SZ = 200 mg KOH/g, Iodzahl = 92.3 g $I_2$/100 g, 91.1 g, 0.325 mol), Sebacinsäure (17.0 g, 0.084 mol), Dimersäure (Radiacid 0977 der Firma Oleon mit ca. 36 Kohlenstoffatomen, Funktionalität = 2.0, SZ = 191 mg KOH/g, 42.6 g, 0.074 mol) und Polyhydroxystearinsäure (SZ = 48 mg KOH/g, 140.1 g, 0.120 mol) wurde unter Rühren auf 240 °C erhitzt und das entstehende Wasser so lange kontinuierlich abdestilliert, bis eine Säurezahl von 5 mg KOH/g erreicht war. Säurezahl: 5 mg KOH/g; Verseifungszahl; 177 mg KOH/g; Hydroxylzahl: 55 mg KOH/g; Veresterungsgrad: 76%.

*Beispiel 2: PGE erfindungsgemäß*

Ein Gemisch aus Polyglycerin (OHZ = 1073 mg KOH/g, 52.3 g, 0.167 mol), Ölsäure (Palmera A1818 der Firma KLK Emmerich GmbH, SZ = 200 mg KOH/g, Iodzahl = 92.3 g $I_2$/100 g, 100.0 g, 0.357 mol) und Polyhydroxystearinsäure (SZ = 52 mg KOH/g, 120.2 g, 0.111 mol) wurde unter Rühren auf 240 °C erhitzt und das entstehende Wasser so lange kontinuierlich abdestilliert, bis eine Säurezahl von 8 mg KOH/g erreicht war. Nach Zugabe von Sebacinsäure (18.3 g, 0.090 mol) und Dimersäure (Radiacid 0977 der Firma Oleon mit ca. 36 Kohlenstoffatomen, Funktionalität = 2.0, SZ = 193.5 mg KOH/g, 38.2 g, 0.066 mol) wurde das Gemisch weiter unter Rühren auf 240 °C erhitzt und das entstehende Wasser so lange kontinuierlich abdestilliert, bis eine Säurezahl von 2 mg KOH/g erreicht war. Säurezahl: 2 mg KOH/g; Verseifungszahl; 181 mg KOH/g; Hydroxylzahl: 48 mg KOH/g; Veresterungsgrad: 79%.

*Beispiel 3: PGE erfindungsgemäß*

Ein Gemisch aus Polyglycerin (OHZ = 1024 mg KOH/g, 63.0 g, 0.170 mol), Sebacinsäure (12.0 g, 0.059 mol) und Dimersäure (Radiacid 0977 der Firma Oleon mit ca. 36 Kohlenstoffatomen, Funktionalität = 2.0, SZ = 193.5 mg KOH/g, 48.2 g, 0.084 mol) wurde unter Rühren auf 240 °C erhitzt und das entstehende Wasser so lange kontinuierlich abdestilliert, bis eine Säurezahl von 11 mg KOH/g erreicht war. Nach Zugabe von Ölsäure (Palmera A1818 der Firma KLK Emmerich GmbH, SZ = 200 mg KOH/g, Iodzahl = 92.3 g $I_2$/100 g, 75.4 g, 0.269 mol) und Polyhydroxystearinsäure (SZ = 42 mg KOH/g, 100.0 g, 0.075 mol) wurde das Gemisch weiter unter Rühren auf 240 °C erhitzt und das entstehende Wasser so lange kontinuierlich abdestilliert, bis eine Säurezahl von 3 mg KOH/g erreicht war. Säurezahl: 3 mg KOH/g; Verseifungszahl; 164 mg KOH/g; Hydroxylzahl: 110 mg KOH/g; Veresterungsgrad: 59%.

*Beispiel 4: PGE erfindungsgemäß*

Ein Gemisch aus Polyglycerin (OHZ = 1136 mg KOH/g, 52.5 g, 0.201 mol), Ölsäure (Palmera A1818 der Firma KLK Emmerich GmbH, SZ = 200 mg KOH/g, Iodzahl = 92.3 g $I_2$/100 g, 90.0 g, 0.321 mol), Sebacinsäure (16.0 g, 0.079 mol) und Dimersäure (Radiacid 0977 der Firma Oleon mit ca. 36 Kohlenstoffatomen, Funktionalität = 2.0, SZ = 191 mg KOH/g, 46.4 g, 0.081 mol) wurde unter Rühren auf 240 °C erhitzt und das entstehende Wasser so lange kontinuierlich abdestilliert, bis eine Säurezahl von 5 mg KOH/g erreicht war. Nach Zugabe von Polyhydroxystearinsäure (SZ = 48 mg KOH/g, 140.4 g, 0.120 mol) wurde das Gemisch weiter unter Rühren auf 240 °C erhitzt und das entstehende Wasser so lange kontinuierlich abdestilliert, bis eine Säurezahl von 2 mg KOH/g erreicht war. Säurezahl: 2 mg KOH/g; Verseifungszahl; 178 mg KOH/g; Hydroxylzahl: 57 mg KOH/g; Veresterungsgrad: 76%.

*Beispiel 5: PGE erfindungsgemäß*

Ein Gemisch aus Polyglycerin (OHZ = 1040 mg KOH/g, 67.6 g, 0.193 mol), Isostearinsäure (94.8 g, 0.325 mol), Sebacinsäure (17.4 g, 0.086 mol), Dimersäure (Radiacid 0977 der Firma Oleon mit ca. 36 Kohlenstoffatomen, Funktionalität = 2.0, SZ = 191 mg KOH/g, 37.3 g, 0.065 mol) und Polyhydroxystearinsäure (SZ = 48 mg KOH/g, 120.0 g, 0.103 mol) wurde unter Rühren auf 240 °C erhitzt und das entstehende Wasser so lange kontinuierlich abdestilliert, bis eine Säurezahl von 3 mg KOH/g erreicht war. Säurezahl: 3 mg KOH/g; Verseifungszahl; 167 mg KOH/g; Hydroxylzahl: 95 mg KOH/g; Veresterungsgrad: 63%.

*Beispiel 6: PGE erfindungsgemäß*

Ein Gemisch aus Polyglycerin (OHZ = 1070 mg KOH/g, 65.0 g, 0.206 mol), Isostearinsäure (91.1 g, 0.312 mol) und Sebacinsäure (16.9 g, 0.084 mol) wurde unter Rühren auf 240 °C erhitzt und das entstehende Wasser so lange kontinuierlich abdestilliert, bis eine Säurezahl von 0.4 mg KOH/g erreicht war. Nach Zugabe von Dimersäure (Radiacid 0977 der Firma Oleon mit ca. 36 Kohlenstoffatomen, Funktionalität = 2.0, SZ = 193.5 mg KOH/g, 35.4 g, 0.062 mol) wurde das Gemisch weiter unter Rühren auf 240 °C erhitzt und das entstehende Wasser so lange kontinuierlich abdestilliert, bis eine Säurezahl von 0.8 mg KOH/g erreicht war. Nach Zugabe von Polyhydroxystearinsäure (SZ = 45 mg KOH/g, 135.0 g, 0.108 mol) wurde das Gemisch weiter unter Rühren auf 240 °C erhitzt und das entstehende Wasser so lange kontinuierlich abdestilliert, bis eine Säurezahl von 1.0 mg KOH/g erreicht war. Säurezahl: 1 mg KOH/g; Verseifungszahl; 171 mg KOH/g; Hydroxylzahl: 93 mg KOH/g; Veresterungsgrad: 65%.

*Beispiel 7: PGE erfindungsgemäß*

Ein Gemisch aus Polyglycerin (OHZ = 1107 mg KOH/g, 50.5 g, 0.179 mol), Ölsäure (Mascid 1318 der Firma ICOF Europe, SZ = 199 mg KOH/g, Iodzahl = 92.7 g $I_2$/100 g, 89.0 g, 0.317 mol), Sebacinsäure (16.6 g, 0.082 mol), Dimersäure (Radiacid 0977 der Firma Oleon mit ca. 36 Kohlenstoffatomen, Funktionalität = 2.0, SZ = 193.5 mg KOH/g, 44.6 g, 0.078 mol) und Polyricinolsäure (SZ = 45 mg KOH/g, 144.1 g, 0.116 mol) wurde unter Rühren auf 240 °C erhitzt und das entstehende Wasser so lange kontinuierlich abdestilliert, bis eine Säurezahl von 2.5 mg KOH/g erreicht war. Säurezahl: 2.5 mg KOH/g; Verseifungszahl; 196 mg KOH/g; Hydroxylzahl: 50 mg KOH/g; Veresterungsgrad: 79%.

*Beispiel 8: PGE erfindungsgemäß*

Ein Gemisch aus Polyglycerin (OHZ = 1113 mg KOH/g, 59.5 g, 0.178 mol), Isostearinsäure (90.3 g, 0.310 mol), Sebacinsäure (15.0 g, 0.074 mol), Dimersäure (Radiacid 0977 der Firma Oleon mit ca. 36 Kohlenstoffatomen, Funktionalität = 2.0, SZ = 193.5 mg KOH/g, 46.2 g, 0.080 mol) und Polyhydroxystearinsäure (SZ = 47 mg KOH/g, 133.1 g, 0.111 mol) wurde unter Rühren auf 240 °C erhitzt und das entstehende Wasser so lange kontinuierlich abdestilliert, bis eine Säurezahl von 1.2 mg KOH/g erreicht war. Säurezahl: 1.2 mg KOH/g; Verseifungszahl; 188 mg KOH/g; Hydroxylzahl: 49 mg KOH/g; Veresterungsgrad: 79%.

*Beispiel 9: PGE erfindungsgemäß*

Ein Gemisch aus Polyglycerin (OHZ = 1107 mg KOH/g, 50.5 g, 0.179 mol), Isostearinsäure (75.0 g, 0.257 mol), Sebacinsäure (14.0 g, 0.069 mol), Dimersäure (Radiacid 0977 der Firma Oleon mit ca. 36 Kohlenstoffatomen, Funktionalität = 2.0, SZ = 193.5 mg KOH/g, 40.0 g, 0.070 mol) und Polyricinolsäure (SZ = 45 mg KOH/g, 154.1 g, 0.124 mol) wurde unter Rühren auf 240 °C erhitzt und das entstehende Wasser so lange kontinuierlich abdestilliert, bis eine Säurezahl von 2.4 mg KOH/g erreicht war. Säurezahl: 2.4 mg KOH/g; Verseifungszahl; 193 mg KOH/g; Hydroxylzahl: 48 mg KOH/g; Veresterungsgrad: 80%.

*Beispiel 10: PGE erfindungsgemäß*

Ein Gemisch aus einem ersten Polyglycerin (OHZ = 1165 mg KOH/g, 25.3 g, 0.104 mol), einem zweiten Polyglycerin (OHZ = 967 mg KOH/g, 27.6 g, 0.058 mol), Ölsäure (Mascid 1318 der Firma ICOF Europe, SZ = 199 mg KOH/g, lodzahl = 92.7 g $I_2$/100 g, 91.1 g, 0.325 mol), Sebacinsäure (17.0 g, 0.084 mol), Dimersäure (Radiacid 0977 der Firma Oleon mit ca. 36 Kohlenstoffatomen, Funktionalität = 2.0, SZ = 193.5 mg KOH/g, 42.6 g, 0.074 mol) und Polyhydroxystearinsäure (SZ = 48 mg KOH/g, 140.1 g, 0.120 mol) wurde unter Rühren auf 240 °C erhitzt und das entstehende Wasser so lange kontinuierlich abdestilliert, bis eine Säurezahl von 5 mg KOH/g erreicht war. Säurezahl: 5 mg KOH/g; Verseifungszahl; 177 mg KOH/g; Hydroxylzahl: 48 mg KOH/g; Veresterungsgrad: 78%.

*Beispiel 11: PGE erfindungsgemäß*

Ein Gemisch aus Polyglycerin (OHZ = 1113 mg KOH/g, 52.3 g, 0.188 mol), Ölsäure (Mascid 1318 der Firma ICOF Europe, SZ = 199 mg KOH/g, lodzahl = 92.7 g $I_2$/100 g, 45.0 g, 0.161 mol), Isostearinsäure (47.9 g, 0.164 mol), Sebacinsäure (17.0 g, 0.084 mol), Dimersäure (Radiacid 0977 der Firma Oleon mit ca. 36 Kohlenstoffatomen, Funktionalität = 2.0, SZ = 191 mg KOH/g, 42.6 g, 0.074 mol) und Polyhydroxystearinsäure (SZ = 48 mg KOH/g, 140.1 g, 0.120 mol) wurde unter Rühren auf 240 °C erhitzt und das entstehende Wasser so lange kontinuierlich abdestilliert, bis eine Säurezahl von 5 mg KOH/g erreicht war. Säurezahl: 5 mg KOH/g; Verseifungszahl; 178 mg KOH/g; Hydroxylzahl: 54 mg KOH/g; Veresterungsgrad: 76%.

*Beispiel 12: PGE mit PHS, Ölsäure, nur kurzkettige Dicarbonsäure, nicht erfindungsgemäß*

Ein Gemisch aus Polyglycerin (OHZ = 1124 mg KOH/g, 48.2 g, 0.184 mol), Ölsäure (Mascid 1318 der Firma ICOF Europe, SZ = 199 mg KOH/g, lodzahl = 92.7 g $I_2$/100 g, 80.1 g, 0.286 mol) und Sebacinsäure (14.9 g, 0.074 mol) wurde unter Rühren auf 240 °C erhitzt und das entstehende Wasser so lange kontinuierlich abdestilliert, bis eine Säurezahl von 1.5 mg KOH/g erreicht war. Nach Zugabe von Polyhydroxystearinsäure (SZ = 49 mg KOH/g, 156.7 g, 0.137 mol) wurde das Gemisch weiter unter Rühren auf 240 °C erhitzt und das entstehende Wasser so lange kontinuierlich abdestilliert, bis eine Säurezahl von 2.5 mg KOH/g erreicht war. Säurezahl: 2.5 mg KOH/g; Verseifungszahl; 187 mg KOH/g; Hydroxylzahl: 81 mg KOH/g; Veresterungsgrad: 69%.

*Beispiel 13: PGE mit PHS, Ölsäure, nur langkettige Dicarbonsäure, nicht erfindungsgemäß*

Ein Gemisch aus Polyglycerin (OHZ = 1124 mg KOH/g, 48.6 g, 0.186 mol), Ölsäure (Mascid 1318 der Firma ICOF Europe, SZ = 199 mg KOH/g, lodzahl = 92.7 g $I_2$/100 g, 90.2 g, 0.322 mol), und Dimersäure (Radiacid 0977 der Firma Oleon mit ca. 36 Kohlenstoffatomen, Funktionalität = 2.0, SZ = 193.5 mg KOH/g, 42.7 g, 0.074 mol) wurde unter Rühren auf 240 °C erhitzt und das entstehende Wasser so lange kontinuierlich abdestilliert, bis eine Säurezahl von 2.3 mg KOH/g erreicht war. Nach Zugabe von Polyhydroxystearinsäure (SZ = 49 mg KOH/g, 118.5 g, 0.103 mol) wurde das Gemisch weiter unter Rühren auf 240 °C erhitzt und das entstehende Wasser so lange kontinuierlich abdestilliert, bis eine Säurezahl von 2.5 mg KOH/g erreicht war. Säurezahl: 2.5 mg KOH/g; Verseifungszahl; 170 mg KOH/g; Hydroxylzahl: 77 mg KOH/g; Veresterungsgrad: 76%.

*Beispiel 14: PGE mit PHS, lang- und kurzkettige Dicarbonsäure, nur gesättigte unverzweigte Fettsäure, nicht erfindungsgemäß*

Ein Gemisch aus Polyglycerin (OHZ = 1124 mg KOH/g, 43 g, 0.164 mol), Stearin- und Palmitinsäure im Verhältnis 46:54 (71 g, 0.263 mol), Sebacinsäure (11.6 g, 0.057 mol) und Dimersäure (Radiacid 0977 der Firma Oleon mit ca. 36 Kohlenstoffatomen, Funktionalität = 2.0, SZ = 193.5 mg KOH/g, 44.4 g, 0.077 mol) wurde unter Rühren auf 240 °C erhitzt und das entstehende Wasser so lange kontinuierlich abdestilliert, bis eine Säurezahl von 5 mg KOH/g erreicht war. Nach Zugabe von Polyhydroxystearinsäure (SZ = 49 mg KOH/g, 130 g, 0.114 mol) wurde das Gemisch weiter unter Rühren auf 240 °C erhitzt und das entstehende Wasser so lange kontinuierlich abdestilliert, bis eine Säurezahl von 2.5 mg KOH/g erreicht war. Säurezahl: 2.5 mg KOH/g; Verseifungszahl; 188 mg KOH/g; Hydroxylzahl: 53 mg KOH/g; Veresterungsgrad: 78%.

*Beispiel 15: Differenzierung der Fähigkeit der Emulsionsstabilisierung der erfindungsgemäßen Beispiele gegen den Stand der Technik*

Die folgenden Beispielemulsionen sollen den Gegenstand der Erfindung näher erläutern ohne ihn auf diese Beispiele einzuschränken.

Alle Konzentrationen in den Anwendungsbeispielen sind in Gewichtsprozent angegeben. Zur Herstellung der Emulsionen wurden dem Fachmann bekannte übliche Homogenisierverfahren eingesetzt.

Die Herstellung der Emulsionen erfolgte entweder mittels Heiß/Kalt- bzw. Kalt/Kalt-Verfahren. Im Heiß/Kalt-Verfahren erfolgte die Herstellung typischerweise so, dass die Ölphase auf 70 - 75°C erwärmt wurde. Anschließend wurde die Wasserphase innerhalb von ca. 2 Minuten in die Ölphase eingerührt. Nach Beendigung des Einrührens wurde kurz homogenisiert. Nach Abkühlen der Emulsion unter 30°C erfolgte eine weitere Homogenisierung für ca. 2 Minuten. Im Kalt/Kalt-Verfahren wurde bei Raumtemperatur innerhalb von ca. 2 Minuten die Wasser- in die Ölphase gerührt und die Emulsion im Anschluss homogenisiert.

Zugabe weiterer Inhaltsstoffe (wie z.B. Konservierungsmittel, Wirkstoffe) erfolgte bevorzugt unterhalb von 40°C und nach dem ersten Homogenisieren. Falls die Rezepturen mit organischen Säuren konserviert wurden, wurde der pH-Wert der Wasserphase auf ca. 5 eingestellt.

Diese Versuche sollen zeigen, dass die erfindungsgemäßen Polyglycerinester Vorteile in Bezug auf Emulsionsstabilität aufweisen. Als Repräsentanten für auf dem Stand der Technik beruhenden polyglycerinbasierten W/O Emulgatoren wurden dabei die nicht erfindungsgemäßen Emulgatoren entsprechend der Beispiele 12 - 14 gewählt.

Zur Überprüfung der Lagerstabilität der Emulsionen wurden diese einen Monat bei Raumtemperatur und 45°C gelagert. Zur Überprüfung der Kältestabilität wurden drei Gefrier-Tau-Zyklen von 25°C/-15°C/25°C durchgeführt. Deutliche Veränderungen im Erscheinungsbild oder der Konsistenz sowie insbesondere Öl- oder Wasserabscheidungen wurden als Kriterien für Instabilität gewichtet. Die angegebenen Emulsionsviskositäten wurde mittels Brookfield RVT, Spindel C, 10 UpM (Creme) bzw. Spindel 5, 10 UpM (Lotion) 3 Tage nach Emulsionsherstellung bei Raumtemperatur bestimmt.

Vergleich der erfindungsgemäßen Emulgatoren nach Beispiel 1, 3, 5 und 7 gegen die nicht erfindungsgemäßen Emulgatoren nach Beispiel 12 - 14 erfolgte in drei verschiedenen Rezepturen: 1 - Lotion (Kalt/Kalt), 2 - Creme (Heiß/Kalt), 3 - Quick-Break Creme (Kalt/Kalt). Diese Systeme sollen zeigen, dass es im Gegensatz zum Stand der Technik, welche durch die Emulgatoren nach Synthesebeispielen 12-14 dargestellt sind, mit den erfindungsgemäßen Emulgatoren möglich ist, Emulsionen über einen großen Viskositätsbereich hinweg zu stabilisieren. Insbesondere Rezeptur 3 stellt aufgrund der großen internen Wasserphase eine extreme Herausforderung für den Emulgator dar.

Lotion

| Rezeptur | 1 |
|---|---|
| Emulgator | 2,0 |
| Bienenwachs | 0,5 |
| Castorwachs | 0,5 |
| Paraffinum Perliquidum | 10,5 |
| Decyl Cocoate[1] | 8,0 |
| Tocopherylacetat | 0,5 |
| Cyclopentasiloxan | 6,0 |
| Natriumchlorid | 0,5 |
| Wasser | Ad 100 |
| Glycerin | 3,0 |
| Phenoxyethanol; Ethylhexylglycerin [2] | 0,7 |

(fortgesetzt)

| Rezeptur | 1 |
|---|---|
| Ethanol | 5,0 |

1) TEGOSOFT® DC (Evonik Industries AG)
2) Euxyl PE 9010 (Schülke & Mayr GmbH)

Creme

| Rezeptur | 2 |
|---|---|
| Emulgator | 2,0 |
| Mineralöl | 17,0 |
| Castorwachs | 0,4 |
| Mikrokristallines Wachs | 0,6 |
| Wasser | Ad 100 |
| Natriumchlorid | 0,5 |
| Harnstoff | 10,0 |
| Phenoxyethanol; Ethylhexylglycerin 2) | 0,7 |

Quick-breaking Creme

| Rezeptur | 3 |
|---|---|
| Emulgator | 0,8 |
| Cetyl Dimethicone 3) | 1,6 |
| Diethylhexyl Carbonate 4) | 4,0 |
| Dimethicone 5) | 1,0 |
| Cyclopentasiloxan | 4,0 |
| Magnesiumstearat | 0,3 |
| Wasser | Ad 100 |
| Propylenglykol | 5,0 |
| Natriumchlorid | 1,0 |
| Methylisothiazolinone, Methylparaben, Ethylparaben; Dipropylene Glycol 6) | 0,8 |

3) ABU® Wax 9801 (Evonik Industries AG)
4) TEGOSOFT® DEC (Evonik Industries AG)
5) ABU® 350 (Evonik Industries AG)
6) Microcare MEM (Thor)

Tabelle 1:

| Formulierung | Emulgator nach Bsp. | Viskosität | Emulsionsstabilität unter Lagerung bei | | |
|---|---|---|---|---|---|
| | | | Raumtemperatur | Wärme | Kälte |
| Rezeptur 1 Lotion | 1 | 14 | Stabil | Stabil | Stabil |
| | 3 | 15 | Stabil | Stabil | Stabil |
| | 5 | 14 | Stabil | Stabil | Stabil |
| | 7 | 12 | Stabil | Stabil | Stabil |
| | 12 | 12 | Stabil | Wasserseparation | Stabil |
| | 13 | 14 | Stabil | Wasserseparation | Stabil |
| | 14 | 17 | Stabil | Stabil | Phasentrennung |
| Rezeptur 2 Creme | 1 | 78 | Stabil | Stabil | Stabil |
| | 3 | 76 | Stabil | Stabil | Stabil |
| | 5 | 61 | Stabil | Stabil | Stabil |
| | 7 | 72 | Stabil | Stabil | Stabil |
| | 12 | 33 | Wasserseparation, Viskositätsabfall | Wasserseparation | Stabil |
| | 13 | 38 | Viskositätsabfall | Wasserseparation | Stabil |
| | 14 | 71 | Stabil | Stabil | Stabil |
| Rezeptur 3 *Quick-breaking* Creme | 1 | 72 | Stabil | Stabil | Stabil |
| | 3 | 72 | Stabil | Stabil | Stabil |
| | 5 | 78 | Stabil | Stabil | Stabil |
| | 7 | 67 | Stabil | Stabil | Stabil |
| | 12 | 54 | Stabil | Wasserseparation | Stabil |
| | 13 | 58 | Stabil | Wasserseparation | Stabil |
| | 14 | 63 | Stabil | Stabil | Wasserseparation |

Wie aus Tabelle 1 zu entnehmen ist, stabilisieren nur die erfindungsgemäßen Emulgatoren die Rezepturen 1-3 in der erforderlichen Art und Weise. Die dem Stand der Technik entsprechenden Emulgatoren nach Synthesebeispielen 12-14 zeigen je nach Rezeptur und Emulgatoraufbau bei den unterschiedlichen Stabilitätskriterien deutliche und nicht akzeptable Schwächen hinsichtlich der Emulsionsstabilisierung. Zusätzlich ist der Zugang zu *quick-breaking* Systemen mit den erfindungsgemäßen Emulgatoren überraschend, da *quick-breaking* Systeme mit Emulgatoren, welche in den Schriften EP 1 683 781, EP 1 500 427 und EP 0 835 862 beansprucht werden, nicht zugänglich sind.

*Beispiel 16: Differenzierung der Sensorik der erfindungsgemäßen Beispiele gegen den Stand der Technik*

Zur Differenzierung der sensorischen Eigenschaften der erfindungsgemäßen Emulgatoren gegen den Stand der Technik wurden die Rezepturen 3a bis 3d hergestellt. Der Einfluss auf das Hautgefühl der Formulierungen wurde durch einen Paneltest untersucht. Sechs Personen applizierten jeweils eine definierte Menge von ca. 25 µL der vier Formulierungen auf ein definiertes Testfeld auf der Innenseite des Unterarms, ohne die Zusammensetzung der Formulierungen zu kennen. Die Formulierungen wurden mithilfe eines Fingers durch kreisende Bewegungen in dem Testfeld verteilt. Es erfolgt eine initialen Beurteilung der sensorischen Eigenschaften. Nun wurde 5 Minuten pausiert und danach das Hautgefühl auf dem Testfeld erneut beurteilt.

| Rezeptur | 3a | 3b | 3c | 3d |
|---|---|---|---|---|
| Emulgator nach Bsp. 1 | 2,5 | - | - | - |

(fortgesetzt)

| Rezeptur | 3a | 3b | 3c | 3d |
|---|---|---|---|---|
| Emulgator nach EP 1 683 781 | - | 2,5 | - | - |
| Emulgator nach EP 1 500 427 | - | - | 2,5 | - |
| Emulgator nach EP 0 835 862 | - | - | - | 2,5 |
| Zinkstearat | 0,5 | 0,5 | 0,5 | 0,5 |
| Ethylhexyl Stearate | 7,0 | 7,0 | 7,0 | 7,0 |
| Isohexadecan | 7,0 | 7,0 | 7,0 | 7,0 |
| Cetearyl Ethylhexanoate | 7,0 | 7,0 | 7,0 | 7,0 |
| Wasser | Ad 100 | Ad 100 | Ad 100 | Ad 100 |
| Natriumchlorid | 0,5 | 0,5 | 0,5 | 0,5 |
| Phenoxyethanol; Ethylhexylglycerin [2] | 0,7 | 0,7 | 0,7 | 0,7 |

Die Ergebnisse der Beurteilung des Hautgefühls sind in Tabelle 2 dargestellt. Es werden diejenigen sensorischen Eigenschaften aufgeführt, deren Ausprägungen sich zwischen den vier Emulsionen unterscheiden.

Tabelle 2:

| Sensorische Eigenschaft | Rezeptur 3a erfindungsgemäß | Rezeptur 3b nicht erfindungsgemäß | Rezeptur 3c nicht erfindungsgemäß | Rezeptur 3d nicht erfindungsgemäß |
|---|---|---|---|---|
| Initiale Absorption | Hohe Absorption | Mittlere Absorption | Mittlere Absorption | Mittlere bis geringe Absorption |
| Öligkeit nach 5 min | Geringe Öligkeit | Mittlere Öligkeit | Mittlere Öligkeit | Mittlere Öligkeit |
| Absorption nach 5 min | Sehr hohe Absorption | Hohe Absorption | Hohe Absorption | Hohe Absorption |

Rezeptur 3a unterscheidet sich von den Rezepturen 3b bis 3d durch eine höhere Absorption, sowohl initial als auch nach 5 min, und durch geringere Öligkeit, was gleichbedeutend mit einem leichteren, besseren Hautgefühl ist. Dies ist insofern für den Fachmann überraschend, da eine Verwendung von zusätzlichen mehrfunktionellen Fettsäuren in Emulgatoren ein höheres Molekulargewicht erzeugt und damit typischerweise ein schwereres, schlechteres Hautgefühl in der Emulsion einhergeht, was sich in erhöhter Öligkeit und verminderter Absorption zeigt.

*Formulierungsbeispiele*

Die folgenden Beispiele sollen zeigen, dass die erfindungsgemäßen Polyglycerinester in einer Vielzahl kosmetischer Formulierungen eingesetzt werden können.

Es ist darüber hinaus mit Hilfe der erfindungsgemäßen Emulgatoren möglich, Pigmente oder Festkörper stabil in Emulsionspräparate einzuarbeiten.

Weiterhin zeigen die Beispiele die gute Kompatibilität mit typischen Coemulgatoren, Ölen, Wachsen und Stabilisatoren sowie die gute Verträglichkeit mit emulsionsbelastenden Inhaltsstoffen wie UV-Filtern, antimikrobiellen Wirkstoffen, Elektrolyten oder kosmetischen Wirkstoffen.

Cooling Body Lotion

| Rezeptur | 4a | 4b |
|---|---|---|
| Emulgator nach Bsp. 1 | 2,0 | 1,5 |
| Polyglyceryl-4 Diisostearate/ Polyhydroxystearate/Sebacate [7] | - | 0,5 |
| Castorwachs | 0,5 | 0,5 |

(fortgesetzt)

| Rezeptur | 4a | 4b |
|---|---|---|
| Bienenwachs | 0,5 | 0,5 |
| Ethylhexyl Stearate [8] | 10,0 | 10,0 |
| Diethylhexyl Carbonate [4] | 8,5 | 8,5 |
| Dimethicone [9] | 6,0 | 6,0 |
| Tocopherylacetate | 0,5 | 0,5 |
| Glycerin | 3,0 | 3,0 |
| Wasser | Ad 100 | Ad 100 |
| Natriumchlorid | 1,0 | 1,0 |
| Ethanol | 20,0 | 20,0 |
| [7] ISOLAN® GPS (Evonik Industries AG) [8] TEGOSOFT® OS (Evonik Industries AG) [9] Belsil DM 5 (Wacker Chemical Corp.) | | |

W/O Creme basierend auf natürlichen Inhaltstoffen

| Rezeptur | 5a | 5b |
|---|---|---|
| Emulgator nach Bsp. 3 | 3,0 | 2,5 |
| Diisostearyl Polyglyceryl-3 Dimer Dilinoleate [10] | - | 0,5 |
| Diethylhexyl Carbonate [4] | 7,0 | 7,0 |
| Oleyl Erucate [11] | 3,0 | 3,0 |
| Mandelöl | 7,0 | 7,0 |
| Sheabutter | 2,0 | 2,0 |
| Cetyl Ricinoleate [12] | 1,0 | 1,0 |
| Bienenwachs | 0,6 | 0,6 |
| Castorwax | 0,4 | 0,4 |
| Glycerin | 5,0 | 5,0 |
| Wasser | Ad 100 | Ad 100 |
| Magnesiumsulfat Heptahydrat | 1,5 | 1,5 |
| Sodium Benzoate, Potassium Sorbate [13] | 0,5 | 0,5 |
| [10] ISOLAN® PDI (Evonik Industries AG) [11] TEGOSOFT® OER (Evonik Industries AG) [12] TEGOSOFT® CR (Evonik Industries AG) [13] Euxyl K 712 (Schülke & Mayr GmbH) | | |

Kalt herstellbare Lotion

| Rezeptur | 6a | 6b | 6c | 6d |
|---|---|---|---|---|
| Emulgator nach Bsp. 2 | 3.0 | 3.0 | 2.5 | 2.5 |
| Polyglyceryl-3 Oleate [14] | - | - | 0.5 | 0.5 |
| Isoamyl Cocoate [15] | 5.0 | 5.00 | 5.0 | 5.0 |
| Diethylhexyl Carbonate [4] | 12.0 | 12.0 | 12.0 | 12.0 |

(fortgesetzt)

| Rezeptur | 6a | 6b | 6c | 6d |
|---|---|---|---|---|
| Phenoxyethyl Caprylate [16] | 4.0 | 4.0 | 4.0 | 4.0 |
| Zinkstearate | 0.5 | 0.5 | 0.5 | 0.5 |
| Wasser | Ad 100 | Ad 100 | Ad 100 | Ad 100 |
| Glycerin | 3.0 | 3.0 | 3.0 | 3.0 |
| Natriumchlorid | 1.5 | 7.0 | 1.5 | 7.0 |
| Phenoxyethanol; Ethylhexylglycerin [2] | 0.7 | 0.7 | 0.7 | 0.7 |
| [14] ISOLAN® GO 33 (Evonik Industries AG) [15] TEGOSOFT® AC (Evonik Industries AG) [16] TEGOSOFT® XC (Evonik Industries AG) | | | | |

Feuchtigkeitsspendende Lotion mit Harnstoff

| Rezeptur | 7a | 7b |
|---|---|---|
| Emulgator nach Bsp. 2 | 2,0 | 1,5 |
| Cetyl PEG/PPG-10/1 Dimethicone [17] | - | 0,5 |
| Mikrokristallines Wachs | 0,5 | 0,5 |
| Castorwachs | 0,5 | 0,5 |
| C12-15 Alkyl Benzoate | 7,5 | 7,5 |
| Oleyl Erucate [11] | 5,0 | 5,0 |
| Ethylhexyl Palmitate [18] | 5,0 | 5,0 |
| Caprylic/Capric Triglyceride | 5,0 | 5,0 |
| Glycerin | 3,0 | 3,0 |
| Harnstoff | 20,0 | 20,0 |
| Magnesiumsulfat Heptahydrat | 1,0 | 1,0 |
| Wasser | Ad 100 | Ad 100 |
| Phenoxyethanol; Ethylhexylglycerin [2] | 0,70 | 0,70 |
| Parfum | 0,10 | 0,10 |
| [17] ABU® EM 180 (Evonik Industries AG) [18] TEGOSOFT® OP (Evonik Industries AG) | | |

W/O Lotion mit seidig-leichtem Hautgefühl

| Rezeptur | 8a | 8b | 8c |
|---|---|---|---|
| Emulgator nach Bsp. 4 | 2,5 | 2,0 | 2,0 |
| Cetyl PEG/PPG-10/1 Dimethicone [19] | - | 0,5 | - |
| Bis-PEG/PPG-14/14 Dimethicone; Dimethicone [20] | - | - | 1,0 |
| Mikrokristallines Wachs | 0,1 | 0,1 | 0,1 |
| Castorwachs | 0,1 | 0,1 | 0,1 |
| Diethylhexyl Carbonate [4] | 11,8 | 11,8 | 11,8 |
| Myristyl Myristate [21] | 1,0 | 1,0 | 1,0 |

(fortgesetzt)

| Rezeptur | 8a | 8b | 8c |
|---|---|---|---|
| Dimethicone [9] | 8,0 | 8,0 | 8,0 |
| Dimethicone [5] | 0,5 | 0,5 | 0,5 |
| Glycerin | 3,0 | 3,0 | 3,0 |
| Magnesiumsulfat Heptahydrat | 1,5 | 1,5 | 1,5 |
| Wasser | Ad 100 | Ad 100 | Ad 100 |
| Benzyl Alcohol; Ethylhexylglycerin; Tocopherol [22] | 0,7 | 0,7 | 0,7 |
| [19] ABU® EM 90 (Evonik Industries AG)<br>[20] ABIL® EM 97 S (Evonik Industries AG)<br>[21] TEGOSOFT® MM (Evonik Industries AG)<br>[22] Euxyl K 900 (Schülke & Mayr GmbH) | | | |

Babypflege

| Rezeptur | 9a | 9b |
|---|---|---|
| Emulgator nach Bsp. 1 | 3,0 | 2,0 |
| Paraffinum Liquidum; Petrolatum; Ozokerite; Glyceryl Oleate; Lanolin Alcohol[23] | - | 1,0 |
| Castorwachs | 0,1 | 0,1 |
| Mikrokristallines Wachs | 0,1 | 0,1 |
| Oleyl Erucate[11] | 1,0 | 1,0 |
| Isoamyl Cocoate[15] | 3,8 | 3,8 |
| Ethylhexyl Palmitate[18] | 1,0 | 1,0 |
| Mandelöl | 1,0 | 1,0 |
| Zinkoxid | 20,0 | 20,0 |
| Glycerin | 3,0 | 3,0 |
| Magnesiumsulfat Heptahydrat | 1,0 | 1,0 |
| Sodium Lactate; Sodium PCA; Glycine; Fructose; Urea; Niacinamide; Inositol; Sodium Benzoate; Lactic Acid[24] | 5,0 | 5,0 |
| Betaine[25] | 3,0 | 3,0 |
| Wasser | Ad 100 | Ad 100 |
| Benzyl Alcohol; Ethylhexylglycerin; Tocopherol [22] | 0,7 | 0,7 |
| [23] PROTEGIN® XN (Evonik Industries AG)<br>[24] LACTIL® (Evonik Industries AG)<br>[25] TEGO® Natural Betaine (Evonik Industries AG) | | |

Fußpflege

| Rezeptur | 10a | 10b |
|---|---|---|
| Emulgator nach Bsp. 5 | 3,0 | 2,5 |
| Petrolatum; Ozokerite; Hydrogenated Castor Oil; Glyceryl Isostearate; Polyglyceryl-3 Oleate[26] | - | 0,5 |
| Castorwachs | 0,1 | 0,1 |

(fortgesetzt)

| Rezeptur | 10a | 10b |
|---|---|---|
| Mikrokristallines Wachs | 0,1 | 0,1 |
| Diethylhexyl Carbonate[4] | 9,0 | 9,0 |
| Ethylhexyl Palmitate[18] | 9,0 | 9,0 |
| Stearyl Heptanoate[27] | 8,8 | 8,8 |
| Glycerin | 3,0 | 3,0 |
| Magnesiumsulfat Heptahydrat | 1,0 | 1,0 |
| Ceramide NP; Ceramide AP; Ceramide EOP; Phytosphingosine; Cholesterol; Sodium Lauroyl Lactylate; Carbomer; Xanthan Gum[28] | 5,0 | 5,0 |
| Betaine[25] | 3,0 | 3,0 |
| Wasser | Ad 100 | Ad 100 |
| Benzyl Alcohol; Ethylhexylglycerin; Tocopherol [22] | 0,7 | 0,7 |
| [26] PROTEGIN® W (Evonik Industries AG)<br>[27] TEGOSOFT® SH (Evonik Industries AG)<br>[28] SK-INFLUX® V (Evonik Industries AG) | | |

Sonnenschutzlotion SPF 30 UVA mit Insektenschutz

| Rezeptur | 11a | 11b |
|---|---|---|
| Emulgator nach Bsp. 3 | 3,0 | 2,0 |
| Polyglyceryl-2 Dipolyhydroxystearate[29] | - | 1,0 |
| Oleyl Erucate[11] | 1,5 | 1,5 |
| Diethylhexyl Carbonate[4] | 1,5 | 1,5 |
| Diethylamino Hydroxybenzoyl Hexyl Benzoate[30] | 5,4 | 5,4 |
| Ethylhexyl Methoxycinnamate | 10,0 | 10,0 |
| Octocrylene | 2,0 | 2,0 |
| Polyacrylamide; C13-14 Isoparaffin; Laureth-7 [31] | 2,1 | 2,1 |
| Ethyl Butylacetylaminopropionate[32] | 4,0 | 4,0 |
| Tocopheryl Acetat | 0,5 | 0,5 |
| Glycerin | 3,0 | 3,0 |
| Ethanol | 0,5 | 0,5 |
| Magnesiumsulfat Heptahydrat | 1,0 | 1,0 |
| Wasser | Ad 100 | Ad 100 |
| Benzyl Alcohol; Ethylhexylglycerin; Tocopherol [22] | 0,7 | 0,7 |
| Parfum | 0,1 | 0,1 |
| [29] Dehymuls PGPH (BASF SE)<br>[30] Uvinul A Plus (BASF SE)<br>[31] Sepigel 305 (Seppic)<br>[32] IR3535 (Merck KGaA) | | |

Sonnenschutzlotion SPF 30 UVA nach Ecocertkriterien

| Rezeptur | 12a | 12b |
|---|---|---|
| Emulgator nach Bsp. 1 | 3,0 | 2,0 |
| Polyglyceryl-3 Polyricinoleate[33] | - | 1,0 |
| Isoamyl Cocoate[15] | 2,0 | 2,0 |
| Decyl Cocoate[1] | 10,0 | 10,0 |
| Isopropyl Palmitate | 10,0 | 10,0 |
| Zinc Oxide[34] | 16,0 | 16,0 |
| Titanium Dioxide [nano]; Alumina; Stearic Acid[35] | 9,0 | 9,0 |
| Wasser | Ad 100 | Ad 100 |
| Glycerin | 3,0 | 3,0 |
| Magnesiumsulfat Heptahydrat | 1,0 | 1,0 |
| Sodium Benzoate, Potassium Sorbate [13] | 0,5 | 0,5 |
| [33] Cithrol PG3PR (Croda Int. Plc)<br>[34] Zinc Oxide PI (Symrise)<br>[35] Eusolex T-S (Merck KGaA) | | |

Sonnenschutzspray SPF 30 UVA

| Rezeptur | 13a | 13b | 14a | 14b |
|---|---|---|---|---|
| Emulgator nach Bsp. 4 | 3,0 | 2,0 | 3,0 | 2,0 |
| Cetyl PEG/PPG-10/1 Dimethicone[19] | | 1,0 | | - |
| Polyglyceryl-4 Diisostearate/ Polyhydroxystearate/Sebacate[7] | | - | | 1,0 |
| Diethylhexyl Carbonate [4] | 13,0 | 13,0 | 11,3 | 11,3 |
| C12-15 Alkyl Benzoate | 13,0 | 13,0 | 11,3 | 11,3 |
| Bis-Ethyl hexyloxyphenol Methoxyphenyl Triazine[36] | 1,0 | 1,0 | 1,5 | 1,5 |
| Butyl Methoxydibenzoylmethane | - | - | 3,0 | 3,0 |
| Ethylhexyl Methoxycinnamate | 5,0 | 5,0 | - | - |
| Octocrylene | - | - | 6,0 | 6,0 |
| Homosalate | - | - | 4,0 | 4,0 |
| Diethylamino Hydroxybenzoyl Hexyl Benzoate[30] | 5,0 | 5,0 | - | - |
| Wasser | Ad 100 | Ad 100 | Ad 100 | Ad 100 |
| Glycerin | 3,0 | 3,0 | 3,0 | 3,0 |
| Magnesiumsulfat Heptahydrat | 1,0 | 1,0 | 1,0 | 1,0 |
| UV-Filterlösung[37] | 15,0 | 15,0 | 15,0 | 15,0 |
| Benzyl Alcohol; Ethylhexylglycerin; Tocopherol[22] | 0,7 | 0,7 | 0,7 | 0,7 |
| [36] Tinosorb S (BASF SE)<br>[37] 20 % Phenylbenzimidazole Sulfonic Acid (Eusolex 232 (Merck KGaA), 8,8% Tris (hydroxymethyl)-aminomethan, Wasser ad 100% | | | | |

Sonnenschutzlotion SPF 50 UVA

| Rezeptur | 15a | 15b | 16a | 16b |
|---|---|---|---|---|
| Emulgator nach Bsp. 4 | 3,0 | 2,5 | 3,0 | 2,5 |
| Cetyl PEG/PPG-10/1 Dimethicone[17] | - | 1,0 | - | 1,0 |
| Mikrokristallines Wachs | 0,3 | 0,3 | 0,3 | 0,3 |
| Castorwachs | 0,3 | 0,3 | 0,3 | 0,3 |
| Diethylhexyl Carbonate [4] | 2,4 | 2,4 | - | - |
| Phenoxyethyl Caprylate[16] | - | - | 3,9 | 3,9 |
| Bis-Ethylhexyloxyphenol Methoxyphenyl Triazine[36] | 6,0 | 6,0 | - | - |
| Diethylamino Hydroxybenzoyl Hexyl Benzoate[30] | 7,0 | 7,0 | 5,0 | 5,0 |
| Butyl Methoxydibenzoylmethane | - | - | 4,5 | 4,5 |
| Ethylhexyl Salicylate | 3,0 | 3,0 | 5,0 | 5,0 |
| Ethylhexyl Methoxycinnamate | 7,0 | 7,0 | - | - |
| Octocrylene | - | - | 9,0 | 9,0 |
| Homosalate | 3,0 | 3,0 | 5,0 | 5,0 |
| Ethylhexyl Triazone[38] | 1,0 | 1,0 | 2,0 | 2,0 |
| Titanium Dioxide; Silica; Dimethicone[39] | 2,0 | 2,0 | 2,0 | 2,0 |
| Wasser | Ad 100 | Ad 100 | Ad 100 | Ad 100 |
| Glycerin | 3,0 | 3,0 | 3,0 | 3,0 |
| Magnesiumsulfat Heptahydrat | 1,5 | 1,5 | 1,5 | 1,5 |
| Benzyl Alcohol; Ethylhexylglycerin; Tocopherol[22] | 0,7 | 0,7 | 0,7 | 0,7 |
| [38] Uvinul T 150 (BASF SE) [39] Parsol TX (DSM Nutritional Products Llc.) | | | | |

Sonnenschutzlotion SPF 50 nach FDA-Kriterien

| Rezeptur | 17a | 17b |
|---|---|---|
| Emulgator nach Bsp. 6 | 2,0 | 1,5 |
| Lauryl PEG-10 Tris(Trimethylsiloxy)silylethyl Dimethicone[40] | - | 0,5 |
| Ethylhexyl Methoxycinnamate; Diethylamino Hydroxybenzoyl Hexyl Benzoate[41] | 7,5 | 7,5 |
| Ethylhexyl Salicylate | 5,0 | 5,0 |
| Homosalate | 15,0 | 15,0 |
| Butyl Methoxydibenzoylmethane | 3,0 | 3,0 |
| Benzophenone-3 | 6,0 | 6,0 |
| Octocrylene | 10,0 | 10,0 |
| Triisostearin | 2,0 | 2,0 |
| Mikrokristallines Wachs | 1,2 | 1,2 |
| Castorwachs | 0,8 | 0,8 |
| Cetyl Dimethicone [3] | 2,0 | 2,0 |

(fortgesetzt)

| Rezeptur | 17a | 17b |
|---|---|---|
| Diethylhexyl Carbonate [4] | 2,0 | 2,0 |
| Wasser | Ad 100 | Ad 100 |
| Natriumchlorid | 1,0 | 1,0 |
| Ethylendiamintetraessigsäure | 0,1 | 0,1 |
| Propylenglycol | 3,0 | 3,0 |
| Phenoxyethanol; Ethylhexylglycerin [2] | 0,7 | 0,7 |
| [40] ES-5300 Formulation Aid (Dow Corning Corp.)<br>[41] Uvinul A+B (BASF SE) | | |

Foundation

| Rezeptur | 18a | 18b | 18c | 18d | 18e | 18f |
|---|---|---|---|---|---|---|
| Emulgator nach Bsp. 2 | 4,5 | 2,5 | 3,0 | 2,5 | 2,0 | 2.0 |
| Bis-(Glyceryl/Lauryl) Glyceryl Lauryl Dimethicone; Caprylic/ Capric Triglyceride[42] | - | 2,0 | - | - | - | - |
| Polyglyceryl-4 Isostearate[43] | - | - | 1,0 | - | - | - |
| Cetyl Diglyceryl Tris(Trimethylsiloxy)silylethyl Dimethicone[44] | - | - | - | 1,0 | - | - |
| Lauryl Polyglyceryl-3 Polydimethylsiloxyethyl Dimethicone[45] | - | - | - | - | 1,0 | - |
| Polyglyceryl-4 Isostearate; Cetyl PEG/PPG-10/1 Dimethicone; Hexyl Laurate[46] | - | - | - | - | - | 2.0 |
| Isoamyl Cocoate[15] | 10,8 | 10,8 | 10,8 | 10,8 | 10,8 | 10,8 |
| Oleyl Erucate[11] | 8,0 | 8,0 | 8,0 | 8,0 | 8,0 | 8,0 |
| Titanium Dioxide, Alumina, Triethoxycaprylylsilane[47] | 4,0 | 4,0 | 4,0 | 4,0 | 4,0 | 4,0 |
| Iron Oxides[48] | 2,1 | 2,1 | 2,1 | 2,1 | 2,1 | 2,1 |
| Nylon-12[49] | 2,0 | 2,0 | 2,0 | 2,0 | 2,0 | 2,0 |
| Cylcopentasiloxane | 3,5 | 3,5 | 3,5 | 3,5 | 3,5 | 3,5 |
| Disteardimonium Hectorite[50] | 1,0 | 1,0 | 1,0 | 1,0 | 1,0 | 1,0 |
| Propylencarbonat | 0,5 | 0,5 | 0,5 | 0,5 | 0,5 | 0,5 |
| Wasser | Ad 100 | Ad 100 | Ad 100 | Ad 100 | Ad 100 | Ad 100 |
| Magnesiumsulfat Hepathydrat | 1,5 | 1,5 | 1,5 | 1,5 | 1,5 | 1,5 |
| Glycerin | 5,0 | 5,0 | 5,0 | 5,0 | 5,0 | 5,0 |
| Creatine[51] | 0,5 | 0,5 | 0,5 | 0,5 | 0,5 | 0,5 |
| Ceteareth-25; Glycerin; Cetyl Alcohol; Behenic Acid; Cholesterol; Ceramide EOP; Ceramide EOS; Ceramide NP; Ceramide NS; Ceramide AP; Caprooyl Phytospingosine; Caprooyl Sphingosine[52] | 1,5 | 1,5 | 1,5 | 1,5 | 1,5 | 1,5 |

(fortgesetzt)

| Rezeptur | 18a | 18b | 18c | 18d | 18e | 18f |
|---|---|---|---|---|---|---|
| Benzyl Alcohol; Ethylhexylglycerin; Tocopherol[22] | 0,7 | 0,7 | 0,7 | 0,7 | 0,7 | 0,7 |

[42] ABU® EM 120 (Evonik Industries AG)
[43] ISOLAN® GI 34 (Evonik Industries AG)
[44] DC-5600 (Dow Corning Corp.)
[45] KF-6105 (Shin-Etsu Chemical Co.)
[46] ABU® WE 09 (Evonik Industries AG)
[47] Hombitan AC360 (Sachtleben)
[48] Sicovit Braun 70 E 172 (Rockwood)
[49] TEGOLON® 12-20 (Evonik Industries AG)
[50] Bentone 38 V CG (Elementis)
[51] TEGO® Cosmo C 100 (Evonik Industries AG)
[52] SKINMIMICS® (Evonik Industries AG)

CC (Color Control) Fluid

| Rezeptur | 19a | 19b | 19c | 19d | 19e | 19f | 19g |
|---|---|---|---|---|---|---|---|
| Emulgator nach Bsp. 10 | 3,0 | 2,0 | 2,5 | 3,0 | 2,5 | 2,0 | 1,0 |
| Polyglyceryl-4 Diisostearate/Polyhydroxystearate/Sebacate [7] | - | 1,0 | - | - | - | - | - |
| Sorbitan Oleate[53] | - | - | 0,5 | - | - | - | - |
| PEG-30 Dipolyhyd roxystearate[54] | - | - | - | 1,0 | - | - | - |
| Polyglyceryl-3 Diisostearate[55] | - | - | - | - | 1,5 | - | - |
| Glyceryl Oleate, Polyglyceryl-3 Polyricinoleate, Olea Europaea (Olive) Oil Unsaponifiables[56] | - | - | - | - | - | 1,0 | - |
| Lauryl PEG-9 Polydimethylsiloxyethyl Dimethicone[57] | - | - | - | - | - | - | 1,0 |
| Ethylhexyl Methoxycinnamate; Diethylamino Hydroxybenzoyl Hexyl Benzoate[41] | 10,0 | 10,0 | 10,0 | 10,0 | 10,0 | 10,0 | 10,0 |
| Cylcopentasiloxane | 3,5 | 3,5 | 3,5 | 3,5 | 3,5 | 3,5 | 3,5 |
| Disteardimonium Hectorite[50] | 1,0 | 1,0 | 1,0 | 1,0 | 1,0 | 1,0 | 1,0 |
| Propylencarbonat | 0,5 | 0,5 | 0,5 | 0,5 | 0,5 | 0,5 | 0,5 |
| Titanium Dioxide, Alumina, Triethoxycaprylylsilane[47] | 4,0 | 4,0 | 4,0 | 4,0 | 4,0 | 4,0 | 4,0 |
| Talc | 2,0 | 2,0 | 2,0 | 2,0 | 2,0 | 2,0 | 2,0 |
| Iron Oxides; Triethoxycaprylylsilane[58] | 0,4 | 0,4 | 0,4 | 0,4 | 0,4 | 0,4 | 0,4 |
| Iron Oxides; Triethoxycaprylylsilane[59] | 0,12 | 0,12 | 0,12 | 0,12 | 0,12 | 0,12 | 0,12 |
| Iron Oxides; Triethoxycaprylylsilane[60] | 0,08 | 0,08 | 0,08 | 0,08 | 0,08 | 0,08 | 0,08 |
| Diethylhexyl Carbonate[4] | 4,0 | 4,0 | 4,0 | 4,0 | 4,0 | 4,0 | 4,0 |
| C12-15 Alkyl Benzoate | 4,0 | 4,0 | 4,0 | 4,0 | 4,0 | 4,0 | 4,0 |
| Isopropyl Palmitate | 4,0 | 4,0 | 4,0 | 4,0 | 4,0 | 4,0 | 4,0 |
| Nylon-12[49] | 2,0 | 2,0 | 2,0 | 2,0 | 2,0 | 2,0 | 2,0 |
| Wasser | Ad 100 | Ad 100 | Ad 100 | Ad 100 | Ad 100 | Ad 100 | Ad 100 |

(fortgesetzt)

| Rezeptur | 19a | 19b | 19c | 19d | 19e | 19f | 19g |
|---|---|---|---|---|---|---|---|
| Glycerin | 5,0 | 5,0 | 5,0 | 5,0 | 5,0 | 5,0 | 5,0 |
| Natriumchlorid | 1,5 | 1,5 | 1,5 | 1,5 | 1,5 | 1,5 | 1,5 |
| Tetrapeptide-30; Glycerin[61] | 0,3 | 0,3 | 0,3 | 0,3 | 0,3 | 0,3 | 0,3 |
| Phenoxyethanol; Methylparaben; Ethylparaben; Propylparaben[62] | 0,7 | 0,7 | 0,7 | 0,7 | 0,7 | 0,7 | 0,7 |

| |
|---|
| [53] TEGO® SMO V (Evonik Industries AG) |
| [54] Arlacel P135 (Croda) |
| [55] Lameform TGI (BASF SE) |
| [56] Plantasens Natural Emulsifier CP5 (Clariant) |
| [57] KF-6038 (Shin-Etsu Chemical Co.) |
| [58] Unipure Yellow LC 182 AS-EM (Sensient) |
| [59] Unipure Red LC 381 AS-EM (Sensient) |
| [60] Unipure Black LC 989 AS-EM (Sensient) |
| [61] TEGO Pep 4-Even (Evonik Industries AG) |
| [62] Phenonip XB (Clariant) |

AP/Deo Spray bzw. Aerosolspray

| Rezeptur | 20a | 20b | 20c | 20d |
|---|---|---|---|---|
| Emulgator nach Bsp. 1 | 3,0 | 2,0 | 3,0 | 2,0 |
| Polyglyceryl-4 Diisostearate/ Polyhydroxystearate/Sebacate [7] | - | 1,0 | - | 1,0 |
| Isopropyl Palmitate | 20,0 | 20,0 | 20,0 | 20,0 |
| Diethylhexyl Carbonate[4] | 7,0 | 7,0 | 7,0 | 7,0 |
| Wasser | Ad 100 | Ad 100 | Ad 100 | Ad 100 |
| Glycerin | 2,0 | 2,0 | 2,0 | 2,0 |
| Aluminum Chlorohydrate (50% aq.) | 30,0 | 30,0 | 30,0 | 30,0 |
| Parfum | 1,0 | 1,0 | 1,0 | 1,0 |
| Treibgas | - | - | Emulsionen 20cd mit Treibgas im Massenverhältnis 5:2 mischen | |

Sonnenschutzaerosol SPF 50 UVA

| Rezeptur | 21a | 21b | 21c | 21d |
|---|---|---|---|---|
| Emulgator nach Bsp. 4 | 4,0 | 4,0 | 4,0 | 4,0 |
| Cetyl PEG/PPG-10/1 Dimethicone[19] | - | - | 1,0 | 1,0 |
| C12-15 Alkyl Benzoate | 10,0 | 8,0 | 10,0 | 8,0 |
| Diethylhexyl Carbonate[4] | 13,0 | 10,0 | 13,0 | 10,0 |
| Bis-Ethylhexyloxyphenol Methoxyphenyl Triazine[36] | 4,0 | 4,0 | 4,0 | 4,0 |
| Diethylamino Hydroxybenzoyl Hexyl Benzoate[30] | 5,0 | 5,0 | 5,0 | 5,0 |
| Ethylhexyl Salicylate | 5,0 | 5,0 | 5,0 | 5,0 |
| Ethylhexyl Methoxycinnamate | 4,0 | 4,0 | 4,0 | 4,0 |

(fortgesetzt)

| Rezeptur | 21a | 21b | 21c | 21d |
|---|---|---|---|---|
| Wasser | Ad 100 | Ad 100 | Ad 100 | Ad 100 |
| Glycerin | 3,0 | 3,0 | 3,0 | 3,0 |
| UV-Filterlösung[37] | 20,0 | 20,0 | 20,0 | 20,0 |
| Magnesiumsulfat Heptahydrat | 1,0 | 1,0 | 1,0 | 1,0 |
| Benzyl Alcohol; Ethylhexylglycerin; Tocopherol[22] | 0,7 | 0,7 | 0,7 | 0,7 |
| Emulsionen 21a-d mit Treibgas im Massenverhältnis 2:1 mischen | | | | |

W/O quick-breaking Creme basierend auf natürlichen Inhaltsstoffen

| Rezeptur | 22 |
|---|---|
| Emulgator nach Bsp. 3 | 1,4 |
| Isoamyl Cocoate[15] | 5,0 |
| Decyl Cocoate[1] | 1,5 |
| Jojobaöl | 2,1 |
| Mandelöl | 1,5 |
| Wasser | Ad 100 |
| Glycerin | 7,0 |
| Zinksulfat Heptahydrat | 1,5 |
| Sodium Benzoate, Potassium Sorbate[13] | 0,5 |

**Patentansprüche**

1. Polyglycerinpartialester erhältlich durch Veresterung eines Polyglycerins mit einer Carbonsäuremischung umfassend:

    a) mindestens eine Polyhydroxycarbonsäure einer Hydroxycarbonsäure aufweisend 8 bis 32, bevorzugt 12 bis 22, besonders bevorzugt 14 bis 18, Kohlenstoffatome,
    b) mindestens eine kurzkettige Dicarbonsäure aufweisend 2 bis 16, bevorzugt 6 bis 14, besonders bevorzugt 8 bis 12, Kohlenstoffatome,
    c) mindestens eine langkettige Dicarbonsäure aufweisend 24 bis 44, bevorzugt 30 bis 40, besonders bevorzugt 34 bis 38, Kohlenstoffatome, und
    d) mindestens eine Fettsäure ausgewählt aus linearen, ungesättigten und verzweigten, gesättigten Fettsäuren aufweisend 14 bis 24, bevorzugt 16 bis 20, Kohlenstoffatome.

2. Polyglycerinpartialester gemäß Anspruch 1, **dadurch gekennzeichnet, dass** das Polyglycerin einen mittleren Kondensationsgrad von 1,5 bis 8, bevorzugt von 2 bis 6, besonders bevorzugt von 3 bis 5, aufweist.

3. Polyglycerinpartialester gemäß Anspruch 1 oder 2, **dadurch gekennzeichnet, dass** die Polyhydroxycarbonsäure ausgewählt ist aus Polyhydroxystearinsäure und Polyricinolsäure.

4. Polyglycerinpartialester gemäß Anspruch 1 oder 2, **dadurch gekennzeichnet, dass** die Polyhydroxycarbonsäure einen mittleren Kondensationsgrad von 1,5 bis 9, bevorzugt von 2 bis 7, besonders bevorzugt von 3 bis 5, aufweist.

5. Polyglycerinpartialester gemäß mindestens einem der vorherigen Ansprüche, **dadurch gekennzeichnet, dass** die kurzkettige Dicarbonsäure ausgewählt ist aus aliphatischen, linearen Dicarbonsäuren, insbesondere Oxalsäure, Malonsäure, Bernsteinsäure, Glutarsäure, Adipinsäure, Pimelinsäure, Korksäure, Azelainsäure, Sebacinsäure, Dodecandisäure, wobei Sebacinsäure besonders bevorzugt ist.

6. Polyglycerinpartialester gemäß mindestens einem der vorherigen Ansprüche, **dadurch gekennzeichnet, dass** die langkettige Dicarbonsäure ausgewählt ist aus solchen, die aus der Dimerisierung von Ölsäure und/oder Linolsäure erhältlich sind.

7. Polyglycerinpartialester gemäß mindestens einem der vorherigen Ansprüche, **dadurch gekennzeichnet, dass** die mindestens eine Fettsäure ausgewählt aus linearen, ungesättigten und verzweigten, gesättigten Fettsäure ausgewählt ist aus Isostearinsäure Undecylensäure, Myristoleinsäure, Palmitoleinsäure, Petroselinsäure, Ölsäure, Elaidinsäure, Vaccensäure, Gadoleinsäure, Icosensäure, Cetoleinsäure, Erucasäure, Nervonsäure, Linolsäure, Alpha-Linolensäure, Gamma-Linolensäure, Calendulasäure, Punicinsäure, Alpha-Elaeostearinsäure, Beta-Elaeostearinsäure, Arachidonsäure, Timnodonsäure, Clupanodonsäure, Cervonsäure, wobei Ölsäure und Isostearinsäure besonders bevorzugt sind.

8. Polyglycerinpartialester gemäß mindestens einem der vorherigen Ansprüche, **dadurch gekennzeichnet, dass** der Veresterungsgrad von 35 bis 95 %, bevorzugt von 40 bis 90 %, besonders bevorzugt von 45 bis 85 % aller im Polyglycerinpartialester enthaltenen OH-Gruppen, einschließlich der in der Polyhydroxycarbonsäure enthaltenen, beträgt.

9. Polyglycerinpartialester gemäß mindestens einem der vorherigen Ansprüche, **dadurch gekennzeichnet, dass** in der Veresterung pro Mol Polyglycerin
0,1 bis 2, bevorzugt 0,3 bis 1, besonders bevorzugt 0,4 bis 0,8, mol a),
0,1 bis 2, bevorzugt 0,2 bis 0,9, besonders bevorzugt 0,3 bis 0,6, mol b),
0,1 bis 2, bevorzugt 0,2 bis 0,9, besonders bevorzugt 0,2 bis 0,5, mol c), und
0,5 bis 4, bevorzugt 0,8 bis 3, besonders bevorzugt 1,2 bis 2,2, mol d)
eingesetzt werden.

10. Polyglycerinpartialester gemäß mindestens einem der vorherigen Ansprüche, **dadurch gekennzeichnet, dass** in der Veresterung das molare Verhältnis von b) zu c)
von 0,2 : 1,0 bis 1,0 : 0,2, bevorzugt
von 0,5 : 1,0 bis 1,0 : 0,5, besonders bevorzugt von
von 0,7 : 1,0 bis 1,0 : 0,7
beträgt.

11. Verfahren zur Herstellung eines Polyglycerinpartialester, umfassend die Veresterung eines Polyglycerins mit

a) mindestens einer Polyhydroxycarbonsäure einer Hydroxycarbonsäure aufweisend 8 bis 32, bevorzugt 12 bis 22, besonders bevorzugt 14 bis 18, Kohlenstoffatome,
b) mindestens einer kurzkettige Dicarbonsäure aufweisend 2 bis 16, bevorzugt 6 bis 14, besonders bevorzugt 8 bis 12, Kohlenstoffatome,
c) mindestens einer langkettige Dicarbonsäure aufweisend 24 bis 44, bevorzugt 30 bis 40, besonders bevorzugt 34 bis 38, Kohlenstoffatome, und
d) mindestens einer Fettsäure ausgewählt aus linearen, ungesättigten und verzweigten, gesättigten Fettsäuren aufweisend 14 bis 24, bevorzugt 16 bis 20, Kohlenstoffatome,

12. Kosmetische oder pharmazeutische Zubereitungen enthaltend mindestens einen Polyglycerinpartialester gemäß mindestens einem der Ansprüche 1 bis 10 und/oder erhältlich nach dem Verfahren gemäß Anspruch 11, insbesondere in einer Menge von 0,5 Gew.-% bis 20 Gew.-%.

13. Kosmetische oder pharmazeutische Zubereitungen gemäß Anspruch 12, **dadurch gekennzeichnet, dass** es sich um quick-break-Emulsionen handelt, welche eine interne wässrige Phase in einer Menge von 80 Gew.-% bis 94,5 Gew.-%, bevorzugt 83

Gew.-% bis 92 Gew.-%,
eine externe Ölphase in einer Menge von 5 Gew.-% bis 20 Gew.-%, bevorzugt 8 Gew.-% bis 17 Gew.-%, und den mindestens einen Polyglycerinpartialester gemäß mindestens einem der Ansprüche 1 bis 10 und/oder erhältlich nach dem Verfahren gemäß Anspruch 11 in einer Menge von 0,5 Gew.-% bis 2,0 Gew.-%, bevorzugt 0,5 Gew.-% bis 1,5 Gew.-%,

enthalten.

14. Verwendung mindestens eines Polyglycerinpartialesters gemäß mindestens einem der Ansprüche 1 bis 10 und/oder erhältlich nach dem Verfahren gemäß Anspruch 11 als W/O-Emulgator, insbesondere in kosmetischen oder pharmazeutischen Zubereitungen.

15. Verwendung mindestens eines Polyglycerinpartialesters gemäß mindestens einem der Ansprüche 1 bis 10 und/oder erhältlich nach dem Verfahren gemäß Anspruch 11 zur Herstellung von *quick-break*-Emulsionen.

**Claims**

1. Polyglycerol partial ester obtainable by esterification of a polyglycerol with a carboxylic acid mixture comprising:

   a) at least one polyhydroxycarboxylic acid of a hydroxycarboxylic acid having 8 to 32, preferably 12 to 22, particularly preferably 14 to 18, carbon atoms,
   b) at least one short-chain dicarboxylic acid having 2 to 16, preferably 6 to 14, particularly preferably 8 to 12, carbon atoms,
   c) at least one long-chain dicarboxylic acid having 24 to 44, preferably 30 to 40, particularly preferably 34 to 38, carbon atoms, and
   d) at least one fatty acid selected from linear, unsaturated and branched, saturated fatty acids having 14 to 24, preferably 16 to 20, carbon atoms.

2. Polyglycerol partial ester according to Claim 1, **characterized in that** the polyglycerol has an average degree of condensation of 1.5 to 8, preferably of 2 to 6, particularly preferably of 3 to 5.

3. Polyglycerol partial ester according to Claim 1 or 2, **characterized in that** the polyhydroxycarboxylic acid is selected from polyhydroxystearic acid and polyricinoleic acid.

4. Polyglycerol partial ester according to Claim 1 or 2, **characterized in that** the polyhydroxycarboxylic acid has an average degree of condensation of 1.5 to 9, preferably of 2 to 7, particularly preferably of 3 to 5.

5. Polyglycerol partial ester according to at least one of the preceding claims, **characterized in that** the short-chain dicarboxylic acid is selected from aliphatic, linear dicarboxylic acids, in particular oxalic acid, malonic acid, succinic acid, glutaric acid, adipic acid, pimelic acid, suberic acid, azelaic acid, sebacic acid, dodecanedioic acid, wherein sebacic acid is particularly preferred.

6. Polyglycerol partial ester according to at least one of the preceding claims, **characterized in that** the long-chain dicarboxylic acid is selected from those obtainable from the dimerization of oleic acid and/or linoleic acid.

7. Polyglycerol partial ester according to at least one of the preceding claims, **characterized in that** the at least one fatty acid selected from linear, unsaturated and branched, saturated fatty acid is selected from isostearic acid, undecylenic acid, myristoleic acid, palmitoleic acid, petroselinic acid, oleic acid, elaidic acid, vaccenic acid, gadoleic acid, icosenoic acid, cetoleic acid, erucic acid, nervonic acid, linoleic acid, alpha-linolenic acid, gamma-linolenic acid, calendulic acid, punicic acid, alpha-elaeostearic acid, beta-elaeostearic acid, arachidonic acid, timnodonic acid, clupanodonic acid, cervonic acid, wherein oleic acid and isostearic acid are particularly preferred.

8. Polyglycerol partial ester according to at least one of the preceding claims, **characterized in that** the degree of esterification is from 35 to 95%, preferably from 40 to 90%, particularly preferably from 45 to 85%, of all OH groups present in the polyglycerol partial ester, including the ones present in the polyhydroxycarboxylic acid.

9. Polyglycerol partial ester according to at least one of the preceding claims, **characterized in that**, in the esterification,

   0.1 to 2, preferably 0.3 to 1, particularly preferably 0.4 to 0.8, mol of a),
   0.1 to 2, preferably 0.2 to 0.9, particularly preferably 0.3 to 0.6, mol of b),
   0.1 to 2, preferably 0.2 to 0.9, particularly preferably 0.2 to 0.5, mol of c), and
   0.5 to 4, preferably 0.8 to 3, particularly preferably 1.2 to 2.2, mol of d)

are used per mole of polyglycerol.

10. Polyglycerol partial ester according to at least one of the preceding claims, **characterized in that**, in the esterification, the molar ratio of b) to c) is
from 0.2 : 1.0 to 1.0 : 0.2, preferably
from 0.5 : 1.0 to 1.0 : 0.5, more preferably
from 0.7 : 1.0 to 1.0 : 0.7.

11. Method for preparing a polyglycerol partial ester, comprising
the esterification of a polyglycerol with

a) at least one polyhydroxycarboxylic acid of a hydroxycarboxylic acid having 8 to 32, preferably 12 to 22, particularly preferably 14 to 18, carbon atoms,
b) at least one short-chain dicarboxylic acid having 2 to 16, preferably 6 to 14, particularly preferably 8 to 12, carbon atoms,
c) at least one long-chain dicarboxylic acid having 24 to 44, preferably 30 to 40, particularly preferably 34 to 38, carbon atoms, and
d) at least one fatty acid selected from linear, unsaturated and branched, saturated fatty acids having 14 to 24, preferably 16 to 20, carbon atoms.

12. Cosmetic or pharmaceutical preparations comprising at least one polyglycerol partial ester according to at least one of Claims 1 to 10 and/or obtainable in accordance with the method according to Claim 11, especially in an amount from 0.5% by weight to 20% by weight.

13. Cosmetic or pharmaceutical preparations according to Claim 12, **characterized in that** they are *quick-break* emulsions containing
an internal aqueous phase in an amount from 80% by weight to 94.5% by weight, preferably from 83% by weight to 92% by weight,

an external oil phase in an amount from 5% by weight to 20% by weight, preferably from 8% by weight to 17% by weight, and
the at least one polyglycerol partial ester according to at least one of Claims 1 to 10 and/or obtainable in accordance with the method according to Claim 11 in an amount from 0.5% by weight to 2.0% by weight, preferably from 0.5% by weight to 1.5% by weight.

14. Use of at least one polyglycerol partial ester according to at least one of Claims 1 to 10 and/or obtainable in accordance with the method according to Claim 11 as W/O emulsifier, especially in cosmetic or pharmaceutical preparations.

15. Use of at least one polyglycerol partial ester according to at least one of Claims 1 to 10 and/or obtainable in accordance with the method according to Claim 11 for preparing *quick-break* emulsions.

**Revendications**

1. Ester partiel de polyglycérine pouvant être obtenu par estérification d'une polyglycérine avec un mélange d'acides carboxyliques comprenant :

a) au moins un poly(acide hydroxycarboxylique) d'un acide hydroxycarboxylique présentant 8 à 32, préférablement 12 à 22, particulièrement préférablement 14 à 18, atomes de carbone,
b) au moins un acide dicarboxylique à chaîne courte présentant 2 à 16, préférablement 6 à 14, particulièrement préférablement 8 à 12, atomes de carbone,
c) au moins un acide dicarboxylique à chaîne longue présentant 24 à 44, préférablement 30 à 40, particulièrement préférablement 34 à 38, atomes de carbone, et
d) au moins un acide gras choisi parmi des acides gras linéaires, insaturés et ramifiés, saturés présentant 14 à 24, préférablement 16 à 20, atomes de carbone.

2. Ester partiel de polyglycérine selon la revendication 1, **caractérisé en ce que** la polyglycérine présente un degré moyen de condensation de 1,5 à 8, préférablement de 2 à 6, particulièrement préférablement de 3 à 5.

3. Ester partiel de polyglycérine selon la revendication 1 ou 2, **caractérisé en ce que** le poly(acide hydroxycarboxylique) est choisi parmi un poly(acide hydroxystéarique) et un poly(acide ricinique).

4. Ester partiel de polyglycérine selon la revendication 1 ou 2, **caractérisé en ce que** le poly(acide hydroxycarboxylique) présente un degré moyen de condensation de 1,5 à 9, préférablement de 2 à 7, particulièrement préférablement de 3 à 5.

5. Ester partiel de polyglycérine selon au moins l'une des revendications précédentes, **caractérisé en ce que** l'acide dicarboxylique à chaîne courte est choisi parmi des acides dicarboxyliques aliphatiques, linéaires, en particulier l'acide oxalique, l'acide malonique, l'acide succinique, l'acide glutarique, l'acide adipique, l'acide pimélique, l'acide subérique, l'acide azélaïque, l'acide sébacique, l'acide dodécanedioïque ; l'acide sébacique étant particulièrement préféré.

6. Ester partiel de polyglycérine selon au moins l'une des revendications précédentes, **caractérisé en ce que** l'acide dicarboxylique à chaîne longue est choisi parmi ceux qui peuvent être obtenus par dimérisation de l'acide oléique et/ou de l'acide linoléique.

7. Ester partiel de polyglycérine selon au moins l'une des revendications précédentes, **caractérisé en ce que** l'au moins un acide gras choisi parmi des acides gras linéaires, insaturés et ramifiés, saturés est choisi parmi l'acide isostéarique, l'acide undécylénique, l'acide myristoléique, l'acide palmitoléique, l'acide pétrosélinique, l'acide oléique, l'acide élaïdique, l'acide vaccénique, l'acide gadoléique, l'acide icosénoïque, l'acide cétoléique, l'acide érucique, l'acide nervonique, l'acide linoléique, l'acide alpha-linolénique, l'acide gamma-linolénique, l'acide calendulaïque, l'acide punicique, l'acide alpha-élaéostéarique, l'acide bêta-élaéostéarique, l'acide arachidonique, l'acide timnodonique, l'acide clupanodonique, l'acide cervonique ; l'acide oléique et l'acide isostéarique étant particulièrement préférés.

8. Ester partiel de polyglycérine selon au moins l'une des revendications précédentes, **caractérisé en ce que** le degré d'estérification est de 35 à 95 %, préférablement de 40 à 90 %, particulièrement préférablement de 45 à 85 % de tous les groupes OH contenus dans l'ester partiel de polyglycérine, y compris ceux contenus dans le poly(acide hydroxycarboxylique).

9. Ester partiel de polyglycérine selon au moins l'une des revendications précédentes, **caractérisé en ce que** dans l'estérification
   0,1 à 2, préférablement 0,3 à 1, particulièrement préférablement 0,4 à 0,8, mole de a),
   0,1 à 2, préférablement 0,2 à 0,9, particulièrement préférablement 0,3 à 0,6, mole de b),
   0,1 à 2, préférablement 0,2 à 0,9, particulièrement préférablement 0,2 à 0,5, mole de c), et
   0,5 à 4, préférablement 0,8 à 3, particulièrement préférablement 1,2 à 2,2, mole de d) sont utilisées par mole de polyglycérine.

10. Ester partiel de polyglycérine selon au moins l'une des revendications précédentes, **caractérisé en ce que** dans l'estérification, le rapport molaire de b) sur c) est
    de 0,2 : 1,0 à 1,0 : 0,2, préférablement de 0,5 : 1,0 à 1,0 : 0,5, particulièrement préférablement
    de 0,7 : 1,0 à 1,0 : 0,7.

11. Procédé pour la préparation d'un ester partiel de polyglycérine, comprenant l'estérification d'une polyglycérine avec

    a) au moins un poly(acide hydroxycarboxylique) d'un acide hydroxycarboxylique présentant 8 à 32, préférablement 12 à 22, particulièrement préférablement 14 à 18, atomes de carbone,
    b) au moins un acide dicarboxylique à chaîne courte présentant 2 à 16, préférablement 6 à 14, particulièrement préférablement 8 à 12, atomes de carbone,
    c) au moins un acide dicarboxylique à chaîne longue présentant 24 à 44, préférablement 30 à 40, particulièrement préférablement 34 à 38, atomes de carbone, et
    d) au moins un acide gras choisi parmi des acides gras linéaires, insaturés et ramifiés, saturés présentant 14 à 24, préférablement 16 à 20, atomes de carbone.

12. Préparations cosmétiques ou pharmaceutiques contenant au moins un ester partiel de polyglycérine selon au moins l'une des revendications 1 à 10 et/ou pouvant être obtenu par le procédé selon la revendication 11, en particulier en une quantité de 0,5 % en poids à 20 % en poids.

**13.** Préparations cosmétiques ou pharmaceutiques selon la revendication 12, **caractérisées en ce que** ce sont des émulsions de type « *quick-break* », qui contiennent une phase aqueuse interne en une quantité de 80 % en poids à 94,5 % en poids, préférablement 83 % en poids à 92 % en poids, une phase huileuse externe en une quantité de 5 % en poids à 20 % en poids, préférablement 8 % en poids à 17 % en poids, et l'au moins un ester partiel de polyglycérine selon au moins l'une des revendications 1 à 10 et/ou pouvant être obtenu par le procédé selon la revendication 11 en une quantité de 0,5 % en poids à 2,0 % en poids, préférablement 0,5 % en poids à 1,5 % en poids.

**14.** Utilisation d'au moins un ester partiel de polyglycérine selon au moins l'une des revendications 1 à 10 et/ou pouvant être obtenu par le procédé selon la revendication 11 en tant qu'émulsifiant eau/huile, en particulier dans des préparations cosmétiques ou pharmaceutiques.

**15.** Utilisation d'au moins un ester partiel de polyglycérine selon au moins l'une des revendications 1 à 10 et/ou pouvant être obtenu par le procédé selon la revendication 11 pour la préparation d'émulsions de type « *quick-break* ».

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0835862 A
- EP 1683781 A
- EP 1500427 A
- WO 2014090615 A
- EP 2319484 A

**In der Beschreibung aufgeführte Nicht-Patentliteratur**

- The Dimer Acids: The chemical and physicalproperties, reactions and applications. Humko Sheffield Chemical. 1975